(19) Europäisches Patentamt
European Patent Office
Office européen des brevets

(11) EP 4 692 078 A1

(12) **EUROPEAN PATENT APPLICATION**
published in accordance with Art. 153(4) EPC

(43) Date of publication:
**11.02.2026 Bulletin 2026/07**

(21) Application number: **24780641.7**

(22) Date of filing: **28.03.2024**

(51) International Patent Classification (IPC):
***C07D 403/14*** *(2006.01)* ***H10K 30/40*** *(2023.01)*
***H10K 30/50*** *(2023.01)* ***H10K 30/86*** *(2023.01)*
***H10K 85/60*** *(2023.01)*

(52) Cooperative Patent Classification (CPC):
**C07D 403/14; H10K 30/40; H10K 30/50; H10K 30/86; H10K 85/60;** Y02E 10/549

(86) International application number:
**PCT/JP2024/012658**

(87) International publication number:
**WO 2024/204541 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.03.2023 JP 2023053716**

(71) Applicant: **Hodogaya Chemical Co., Ltd.**
**Tokyo, 105-0021 (JP)**

(72) Inventors:
- **HAYASHI, Yuichiro**
**Tokyo 105-0021 (JP)**
- **TAKAHASHI, Hideaki**
**Tokyo 105-0021 (JP)**
- **SATO, Hiroshi**
**Tokyo 105-0021 (JP)**
- **ITO, Toshiaki**
**Tokyo 105-0021 (JP)**
- **SHIMIZU, Yuka**
**Tokyo 105-0021 (JP)**

(74) Representative: **dompatent**
**Partnerschaft von Patentanwälten und Rechtsanwälten mbB**
**Deichmannhaus am Dom**
**Bahnhofsvorplatz 1**
**50667 Köln (DE)**

(54) **COMPOUND, HOLE TRANSPORT MATERIAL, AND PHOTOELECTRIC CONVERSION ELEMENT USING SAME**

(57) A photoelectric conversion device that uses a compound represented by the general formula (1) in the hole transport layer has excellent heat resistance. $R^1$ is an aromatic hydrocarbon group, a heterocyclic group, etc., and $R^2$ to $R^{29}$ each are a hydrogen atom, an alkoxy group, etc.

EP 4 692 078 A1

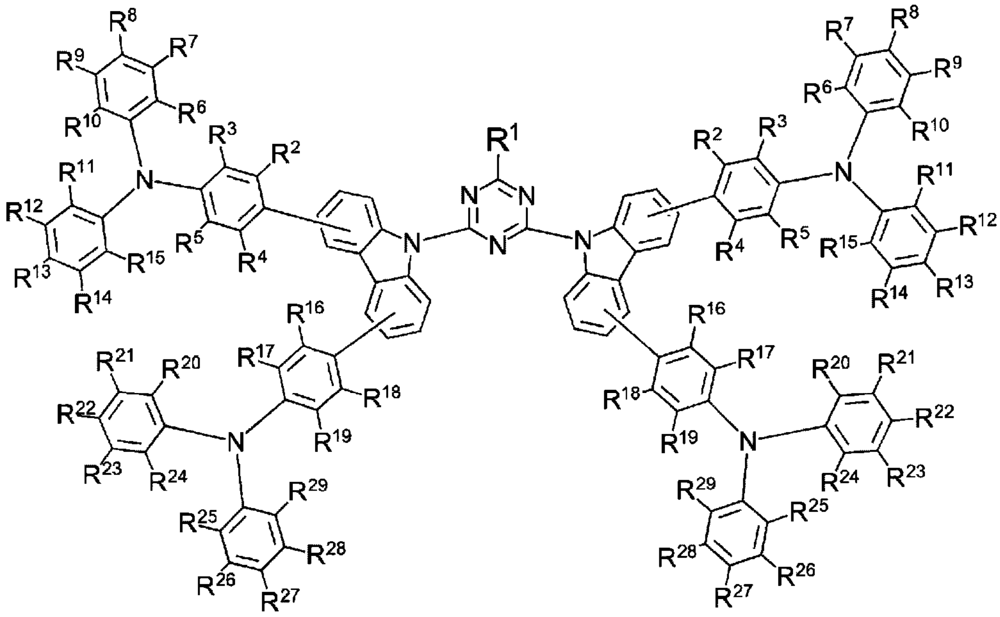

(1)

## Description

### Technical Field

The invention relates to compounds, hole transport materials, and photoelectric conversion devices using them.

### Background Art

In recent years, solar power generation has been attracting attention as a clean energy source, and the development of solar cells has been active. Among them, the development of solar cells using perovskite materials in the photoelectric conversion layer (hereinafter referred to as perovskite solar cells) has attracted attention as a next-generation type solar cell that can be manufactured at low cost in a solution process (e.g., PTL 1, NPLs 1-2).

Perovskite solar cells often use hole transport materials in the device. The purposes of use are (1) to improve the photoelectric conversion efficiency by enhancing the function to selectively transport holes, and (2) to protect perovskite materials that are susceptible to moisture and oxygen by bonding to the perovskite photoelectric conversion layer (e.g., NPL 3). Spiro-OMeTAD [comparative compound (B-1) listed below], a spirobifluorene-type organic compound, is often used as a standard hole transport material, but there are few reports of hole transport materials that contribute higher photoelectric conversion properties than that material. In addition, since photoelectric conversion devices are expected to be used outdoors under sunlight irradiation, high heat resistance is required.

### Citation List

### Patent Literature

PTL 1: WO2017/104792

### Non Patent Literature

NPL 1: J. Am. Chem. Soc., 2009, 131, pp. 6050-6051
NPL 2: Science, 2012, 388, pp. 643-647
NPL 3: Chem. Sci., 2019, 10, pp. 6748-6769

## Summary of Invention

### Technical Problem

The problem that the invention is to solve is to provide a compound useful as a hole transport material for a photoelectric conversion device capable of efficiently extracting electric current, and a photoelectric conversion device and a solar cell with high heat resistance using the compound for the hole transport layer.

### Solution to Problem

In order to solve the above problem, the inventors have assiduously studied the improvement of photoelectric conversion characteristics and have found that, planning, developing and using a compound having a specific structure as a hole transport layer in a photoelectric conversion device, a photoelectric conversion device and a perovskite solar cell that have good photoelectric conversion characteristics and exhibit high heat resistance can be obtained. Specifically, the invention is summarized as follows.

1. A compound represented by the following general formula (1).

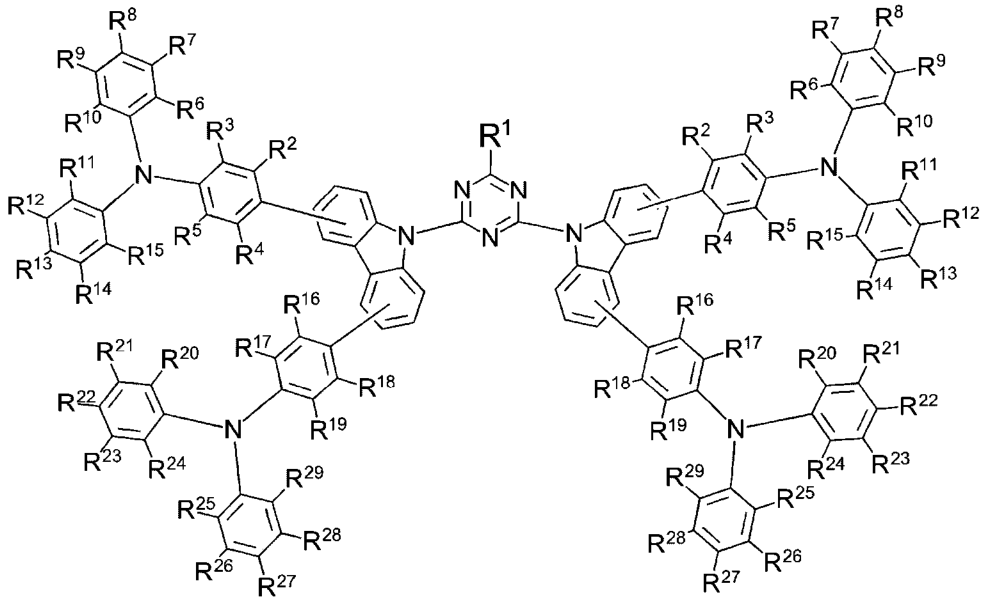

(1)

wherein

$R^1$ represents a hydrogen atom, a halogen atom, a hydroxyl group,
a linear or branched alkyl group with 1 to 20 carbon atoms, which may have a substituent,
a linear or branched alkenyl group with 2 to 20 carbon atoms, which may have a substituent,
a linear or branched alkoxy group with 1 to 20 carbon atoms, which may have a substituent,
an aryloxy group with 6 to 30 carbon atoms, which may have a substituent,
an amino group with 0 to 60 carbon atoms, which may have a substituent,
a thio group with 0 to 20 carbon atoms, which may have a substituent,
a monovalent aromatic hydrocarbon group with 6 to 30 carbon atoms, which may have a substituent, or
a monovalent heterocyclic group with 5 to 30 ring-forming atoms, which may have a substituent,
$R^2$ to $R^{29}$ each independently represent:

a hydrogen atom,
a linear or branched alkyl group with 1 to 20 carbon atoms, which may have a substituent,
a linear or branched alkenyl group with 2 to 20 carbon atoms, which may have a substituent,
a cycloalkyl group with 3 to 12 carbon atoms, which may have a substituent,
a linear or branched alkoxy group with 1 to 20 carbon atoms, which may have a substituent,
an amino group with 0 to 20 carbon atoms, which may have a substituent,
a thio group with 0 to 20 carbon atoms, which may have a substituent, and
$R^{10}$ and $R^{11}$, and $R^{24}$ and $R^{25}$ each may bond to each other to form a ring.

2. In the above general formula (1), $R^1$ is a halogen atom, an amino group with 0 to 60 carbon atoms, which may have a substituent, an aromatic hydrocarbon group with 6 to 20 carbon atoms, which may have a substituent, or a monovalent heterocyclic group with 5 to 30 ring-forming atoms, which may have a substituent.
3. In the above general formula (1), at least one of $R^6$ to $R^{15}$ and $R^{20}$ to $R^{29}$ is a linear or branched alkoxy group with 1 to 20 carbon atoms, which may have a substituent.
4. In the above general formula (1), neither $R^{10}$ and $R^{11}$ nor $R^{24}$ and $R^{25}$ bond to each other to form a ring.
5. A hole transport material containing the compound described above.
6. The hole transport material described above is for the hole transport layer of a photoelectric conversion device.
7. A photoelectric conversion device having a hole transport layer containing the compound described above.
8. A solar cell using the photoelectric conversion device described above.

Advantageous Effects of Invention

According to the compound of the invention and the hole transport layer using the compound, photoelectric conversion devices and perovskite solar cells with high heat resistance can be obtained.

Brief Description of Drawings

[Fig. 1] Fig. 1 is a schematic cross-sectional view of a configuration of the photoelectric conversion device in Examples of the invention and Comparative Example.

Description of Embodiments

The contents of the invention will be described in detail below. The constitutional elements may be described below with reference to representative embodiments and specific examples of the invention, but the invention is not limited to the embodiments and the specific examples. In the description herein, a numerical range expressed as "to" means a range that includes the numerical values described before and after "to" as the lower limit and the upper limit. A part or all of the hydrogen atoms existing in the compound represented by the general formula (1) and in the groups represented by $R^1$ to $R^{29}$ may be substituted with deuterium atoms.

In this description, "transparent" and "translucent" mean that the transmittance of light used for photoelectric conversion is 50% or more, e.g., 80% or more, 90% or more, or 99% or more. The light transmittance can be measured with a UV/visible light spectrophotometer.

The following are specific descriptions of the compounds represented by the general formula (1) of the invention, but the invention is not to be construed as limited by these specific descriptions.

In the general formula (1), $R^1$ represents a hydrogen atom, a halogen atom, a hydroxyl group,

a linear or branched alkyl group with 1 to 20 carbon atoms, which may have a substituent,
a linear or branched alkenyl group with 2 to 20 carbon atoms, which may have a substituent,
a linear or branched alkoxy group with 1 to 20 carbon atoms, which may have a substituent,
an aryloxy group with 6 to 30 carbon atoms, which may have a substituent,
an amino group with 0 to 60 carbon atoms, which may have a substituent,
a thio group with 0 to 20 carbon atoms, which may have a substituent,
an aromatic hydrocarbon group with 6 to 30 carbon atoms, which may have a substituent, or
a heterocyclic group with 5 to 30 ring-forming atoms, which may have a substituent.

In the general formula (1), examples of a "halogen atom" represented by $R^1$ include a fluorine atom, a chlorine atom, a bromine atom and an iodine atom.

In the general formula (1), the number of carbon atoms in the "linear or branched alkyl group with 1 to 20 carbon atoms" in the "linear or branched alkyl group with 1 to 20 carbon atoms, which may have a substituent" represented by $R^1$ is selected from a range of 1 to 20, for example, from 1 to 12, and for example, it may be selected from a range of 1 to 6. Specifically, examples of the "linear or branched alkyl group with 1 to 20 carbon atoms" include a methyl group, an ethyl group, a n-propyl group, an isopropyl group, a n-butyl group, an isobutyl group, a s-butyl group, a t-butyl group, a n-pentyl group, an isopentyl group, a n-hexyl group, a 2-ethylhexyl group, a heptyl group, an octyl group, an isooctyl group, a nonyl group, and a decyl group.

In the general formula (1), the number of carbon atoms in the "linear or branched alkenyl group with 2 to 20 carbon atoms" in the "linear or branched alkenyl group with 2 to 20 carbon atoms, which may have a substituent" represented by $R^1$ is selected from a range of 2 to 20, for example, from 2 to 12, and for example, it may be selected from a range of 2 to 6. Specifically, examples of the "linear or branched alkenyl group with 2 to 20 carbon atoms" include an ethenyl group (vinyl group), a 1-propenyl group, a 2-propenyl group (allyl group), a 1-methylethenyl group, a 1-butenyl group, a 2-butenyl group, a 1-pentenyl group, a 1-hexenyl group, a 2-methyl-1-propenyl group, a 2-methyl-2-propenyl group, a 1-ethylethenyl group, and a linear or branched alkenyl group with 2 to 20 carbon atoms formed by bonding 2 or more these alkenyl groups.

In the general formula (1), the number of carbon atoms in the "linear or branched alkoxy group with 1 to 20 carbon atoms" in the "linear or branched alkoxy group with 1 to 20 carbon atoms, which may have a substituent" represented by $R^1$ is selected from a range of 1 to 20, for example, from 1 to 12, and for example, it may be selected from a range of 1 to 6. Specifically, examples of the "linear or branched alkoxy group with 1 to 20 carbon atoms" include a methoxy group, an ethoxy group, a propoxy group, a n-butoxy group, a n-pentyloxy group, a n-hexyloxy group, a heptyloxy group, an octyloxy group, a nonyloxy group, a decyloxy group, an isopropoxy group, an isobutoxy group, a s-butoxy group, a t-butoxy group, an isooctyloxy group, and a t-octyloxy group.

In the general formula (1), regarding the description and specific examples of the aryl group that bonds to the oxy group of the "aryloxy group with 6 to 30 carbon atoms" in the "aryloxy group with 6 to 30 carbon atoms, which may have a substituent" represented by $R^1$, reference may be made to the description relating to the "monovalent aromatic hydrocarbon group with 6 to 30 carbon atoms" mentioned below. Specifically, examples of the "aryloxy group with 6 to 30 carbon atoms" include a phenoxy group, a tolyloxy group, a biphenylyloxy group, a terphenyloxy group, a naphthyloxy group, an anthryloxy group, a phenanthryloxy group, a fluorenyloxy group, and an indenyloxy group.

In the general formula (1), the "amino group with 0 to 60 carbon atoms" in the "amino group with 0 to 60 carbon atoms, which may have a substituent" represented by $R^1$ may be an unsubstituted amino group, or may be a monosubstituted amino group or a disubstituted amino group. Examples of the substituent of the substituted amino group includes an alkyl group, an aryl group, and an acyl group. Hydrogen atoms of these groups may be substituted with a substituent selected from the following Substituent Group A. Regarding the description and specific examples of the alkyl group and the alkyl group that constitutes the acyl group, reference can be made to the description relating to the "linear or branched alkyl group with 1 to 20 carbon atoms" mentioned above, and regarding the description and specific examples of the aryl group, reference can be made to the description relating to the "monovalent aromatic hydrocarbon group with 6 to 30 carbon atoms" to be mentioned below. The two substituents bonding to the nitrogen atom of the disubstituted amino group do not bond to each other to form a cyclic structure, and groups having such a cyclic structure shall be included in the "heterocyclic group" to be mentioned below in the invention. The number of carbon atoms of the "amino group with 0 to 60 carbon atoms" is selected from a range of 0 to 60, and, for example, may be selected from a range of 0 to 30, for example, from a range of 2 to 11, and for example, from a range of 12 to 24. Specifically, examples of the "amino group with 0 to 60 carbon atoms" include an unsubstituted amino group ($-NH_2$), as a monosubstituted amino group, a methylamino group, an ethylamino group, an acetylamino group and a phenylamino group, and as a disubstituted amino group, a dialkylamino group such as a dimethylamino group and a diethylamino group, a diarylamino group such as a diphenylamino group, and an acetylphenylamino group. In one aspect of the invention, the "amino group with 0 to 60 carbon atoms, which may have a substituent" is an amino group with 0 to 60 carbon atoms, which may be substituted with an alkyl group or an aryl group.

In the general formula (1), the "thio group with 0 to 20 carbon atoms" in the "thio group with 0 to 20 carbon atoms, which may have a substituent" represented by $R^1$ may be an unsubstituted thio group (thiol group, -SH), or may be a substituted thio group in which the hydrogen atom of the thiol group is substituted with a substituent. The number of carbon atoms of the substituted thio group is preferably within a range of 1 to 18, and, for example, may be within a range of 1 to 12, and for example, within a range of 1 to 6. Examples of the substituent for the substituted thio group includes an alkyl group and an aryl group, in which the hydrogen atom may be substituted with a substituent selected from the following Substituent Group A. Regarding the description and specific examples of the alkyl group of a substituent for the thio group, reference can be made to the description relating to the "linear or branched alkyl group with 1 to 20 carbon atoms" mentioned above, and regarding the description and specific examples of the aryl group, reference can be made to the description relating to the "monovalent aromatic hydrocarbon group with 6 to 30 carbon atoms" to be mentioned below. Typical examples of the substituted thio group include a linear or branched alkylthio group with 1 to 20 carbon atoms, which may have a substituent, and an arylthio group with 6 to 30 carbon atoms, which may have a substituent. Specifically, examples of the "thio group with 0 to 20 carbon atoms" include an unsubstituted thio group (thiol group, -SH), an alkylthio group (e.g., a methylthio group, an ethylthio group, a propylthio group), an arylthio group (e.g., a phenylthio group, a biphenylthio group).

In the general formula (1), the aromatic ring that constitutes the "monovalent aromatic hydrocarbon group with 6 to 30 carbon atoms" in the "monovalent aromatic hydrocarbon with 6 to 30 carbon atoms, which may have a substituent" represented by $R^1$ may be a monocyclic ring, or may be a fused ring formed by fusing 2 or more rings, or may be a linked ring formed by linking 2 or more ring via a single bond. In the case of a fused ring, the number of the fusing rings is, for example 2 to 6, and is, for example, 2 to 4. In one aspect of the invention, the aromatic ring contains 2 or more rings. In the case of a fused ring, it forms a "fused polycyclic aromatic group". In the case of a linked ring, the number of the linking rings is, for example 2 to 6, and is, for example, 2 to 4. The number of carbon atoms to form the aromatic ring is selected from a range of 6 to 30, and, for example, may be selected from a range of 6 to 22, or from a range of 6 to 18, and, for example, may be selected from a range of 6 to 14, or 6 to 10. Specifically, examples of the "monovalent aromatic hydrocarbon group with 6 to 30 carbon atoms" include a phenyl group, a biphenyl group, a terphenyl group, a naphthyl group, an anthracenyl group (anthryl group), a phenanthryl group, a fluorenyl group, an indenyl group, a pyrenyl group, a perylenyl group, a fluoranthenyl group, and a triphenylenyl group.

In the general formula (1), the heterocyclic ring that constitutes the "monovalent heterocyclic group with 5 to 30 ring-forming atoms" in the "monovalent heterocyclic group with 5 to 30 ring-forming atoms, which may have a substituent" represented by $R^1$ may be a monocyclic ring, or may be a fused ring formed by fusing two or more rings. In the case of a fused ring, the number of the fusing rings is, for example 2 to 6, and is, for example, 2 to 4. The heterocyclic ring may be an aromatic heterocyclic ring, or an aliphatic heterocyclic ring. In one aspect of the invention, the heterocyclic ring contains 2 or more rings. Examples of the heteroatom constituting the heterocyclic ring include a nitrogen atom, an oxygen atom, and a sulfur atom. The number of ring-forming atoms of the aromatic heterocyclic ring is selected from a range of 5 to 30, and may be selected, for example, from a range of 5 to 18. Specifically, examples of the aromatic heterocyclic group in the "monovalent heterocyclic group with 5 to 30 ring-forming atoms" include a pyridyl group, a pyrimidinyl group, a triazinyl group, a thienyl group, a furyl group (furanyl group), a pyrrolyl group, an imidazolyl group, a pyrazolyl group, a triazolyl group, a quinolyl group, an isoquinolyl group, a naphthyridinyl group, an acridinyl group, a phenanthrolinyl group, a benzofuranyl group, a benzothienyl group, an oxazolyl group, an indolyl group, a carbazolyl group, a benzoxazolyl group, a thiazolyl group, a benzothiazolyl group, a quinoxalinyl group, a benzimidazolyl group, a pyrazolyl group, a dibenzofuranyl

group, a dibenzothienyl group, and a carbonylyl group. The bonding position of these groups is not specifically limited, and for example, the pyridyl group may be any of a 2-pyridyl group, a 3-pyridyl group, or a 4-pyridyl group (for example, a 4-pyridyl group may be selected). In one aspect of the invention, the aromatic heterocyclic group bonds via a carbon atom. In one aspect of the invention, the aromatic heterocyclic group bonds via a nitrogen atom. Specifically, examples of the aliphatic heterocyclic group of the "monovalent heterocyclic group with 5 to 30 ring-forming atoms" include a group bonding via a nitrogen atom, such as a morpholino group, a pyrrolidino group, a piperidino group, or a piperazino group, and a group bonding via a carbon atom such as a tetrahydrofuryl group or a tetrahydrothienyl group. In one aspect of the invention, the "monovalent heterocyclic group with 5 to 30 ring-forming atoms, which may have a substituent" that $R^1$ can take is a monovalent aromatic heterocyclic group with 5 to 30 ring-forming atoms, which bonds via a nitrogen atom and which may have a substituent, or a monovalent aliphatic heterocyclic group with 5 to 30 ring-forming atoms, which may have a substituent. For example, the "monovalent heterocyclic group with 5 to 30 ring-forming atoms, which may have a substituent" that $R^1$ can take is a monovalent aromatic heterocyclic group with 5 to 30 ring-forming atoms, which may have a substituent and which bonds via a nitrogen atom, and the group can be divided into a substituted or unsubstituted carbazol-9-yl group and the other groups. For example, the "monovalent heterocyclic group with 5 to 30 ring-forming atoms, which may have a substituent" that $R^1$ can take is a monovalent aliphatic heterocyclic group with 5 to 30 ring-forming atoms, which may have a substituent, and is specifically a monovalent aliphatic heterocyclic group with 5 to 30 ring-forming atoms, which bonds via a nitrogen atom and which may have a substituent.

Specifically, examples of the "substituent" in the "linear or branched alkyl group with 1 to 20 carbon atoms, which may have a substituent", the "linear or branched alkenyl group with 2 to 20 carbon atoms, which may have a substituent", the "linear or branched alkoxy group with 1 to 20 carbon atoms, which may have a substituent", the "aryloxy group with 6 to 30 carbon atoms, which may have a substituent", the "amino group with 1 to 60 carbon atoms, which may have a substituent", the "thio group with 0 to 20 carbon atoms, which may have a substituent", the "monovalent aromatic hydrocarbon group with 6 to 30 carbon atoms, which may have a substituent" or the "monovalent heterocyclic group with 5 to 30 ring-forming atoms, which may have a substituent", represented by $R^1$ in the general formula (1), include a halogen atom, such as a fluorine atom, a chlorine atom, a bromine atom, and an iodine atom; a cyano group; a hydroxyl group; a nitro group; a nitroso group; a carboxyl group; a phosphate group; a thioxo group (>C=S); a trimethylsilyl group; a carboxylic acid ester group such as a methyl ester group and an ethyl ester group; a linear or branched alkyl group with 1 to 18 carbon atoms, such as a methyl group, an ethyl group, a n-propyl group, an isopropyl group, a n-butyl group, an isobutyl group, a s-butyl group, a t-butyl group, a n-pentyl group, an isopentyl group, a n-hexyl group, a 2-ethylhexyl group, a heptyl group, an octyl group, an isooctyl group, a nonyl group, a decyl group; a linear or branched alkenyl group with 2 to 18 carbon atoms, such as an ethenyl group (vinyl group), a 1-propenyl group, a 2-propenyl group (allyl group), a 1-butenyl group, a 2-butenyl group, a 1-pentenyl group, a 1-hexenyl group, a 2-methyl-1-propenyl group, a 2-methyl-2-propenyl group, a 1-ethylethenyl group; a linear or branched alkoxy group with 1 to 18 carbon atoms, such as a methoxy group, an ethoxy group, a propoxy group, a t-butoxy group, a pentyloxy group, and a hexyloxy group; an aromatic hydrocarbon group with 6 to 30 carbon atoms, such as a phenyl group, a naphthyl group, an anthryl group, a phenanthryl group, and a pyrenyl group; a heterocyclic group with 5 to 20 ring-forming atoms, such as a pyridyl group, a pyrimidinyl group, a triazinyl group, a thienyl group, a furyl group (furanyl group), a pyrrolyl group, an imidazolyl group, a pyrazolyl group, a triazolyl group, a quinolyl group, an isoquinolyl group, a naphthyridinyl group, an acridinyl group, a phenanthrolinyl group, a benzofuranyl group, a benzothienyl group, an oxazolyl group, an indolyl group, a carbazolyl group, a benzoxazolyl group, a thiazolyl group, a benzothiazolyl group, a quinoxalinyl group, a benzimidazolyl group, a pyrazolyl group, a dibenzofuranyl group, a dibenzothienyl group, and a carbonylyl group; an unsubstituted amino group ($-NH_2$); a substituted amino group with 1 to 18 carbon atoms which is a monosubstituted amino group, such as an alkylamino group (e.g., a methylamino group), an acetylamino group, and an arylamino group (e.g., a phenylamino group), or a disubstituted amino group such as a diethylamino group, a diphenylamino group, and an acetylphenylamino group; an unsubstituted thio group (thiol group: -SH); and a substituted thio group with 1 to 18 carbon atoms, such as a methylthio group, an ethylthio group, a propylthio group, a phenylthio group, and a biphenylthio group; these substituents are referred to as "Substituent Group A" herein. Each group herein may contain only one, or two or more of these "substituents", and when two or more of these "substituents" are contained, they may be the same as or different from each other. Hydrogen atoms of these substituents that constitute Substituent Group A may be further substituted with any substituent selected from Substituent Group A.

In one aspect of the invention, $R^1$ in the general formula (1) is a hydrogen atom, a halogen atom (e.g., chlorine atom), a hydroxyl group, a linear or branched alkyl group with 1 to 20 carbon atoms, which may have a substituent, a linear or branched alkoxy group with 1 to 20 carbon atoms, which may have a substituent, an aryloxy group with 6 to 30 carbon atoms, which may have a substituent, an amino group with 0 to 60 carbon atoms, which may have a substituent (e.g., an alkyl group or an aryl group), a thiol group, an alkylthio group with 1 to 20 carbon atoms, which may have a substituent, a monovalent aromatic hydrocarbon group with 6 to 30 carbon atoms, which may have a substituent, or a monovalent heterocyclic group with 5 to 30 ring-forming atoms, which may have a substituent (preferably a monovalent aromatic heterocyclic group with 5 to 30 ring-forming atoms, which bonds via a carbon atom and which may have a substituent, or a monovalent aliphatic heterocyclic group with 5 to 30 ring-forming atoms, which may have a substituent and which bonds

via a nitrogen atom).

In one aspect of the invention, $R^1$ in the general formula (1) is a halogen atom (e.g., chlorine atom), an amino group with 0 to 60 carbon atoms that may have a substituent, or a monovalent aromatic hydrocarbon group with 6 to 30 carbon atoms that may have a substituent.

In one aspect of the invention, $R^1$ in the general formula (1) is a halogen atom (e.g., chlorine atom), a monovalent heterocyclic group with 5 to 30 ring-forming atom that may have a substituent, or a monovalent aromatic hydrocarbon group with 6 to 30 carbon atoms that may have a substituent.

In the general formula (1), $R^2$ to $R^{29}$ each independently represent

a hydrogen atom,
a linear or branched alkyl group with 1 to 20 carbon atoms, which may have a substituent,
a linear or branched alkenyl group with 2 to 20 carbon atoms, which may have a substituent,
a cycloalkyl group with 3 to 12 carbon atoms, which may have a substituent,
a linear or branched alkoxy group with 1 to 20 carbon atoms, which may have a substituent,
an amino group with 0 to 20 carbon atoms, which may have a substituent, or
a thio group with 0 to 20 carbon atoms, which may have a substituent.

In the general formula (1), examples of the "linear or branched alkyl group with 1 to 20 carbon atoms" in the "linear or branched alkyl group with 1 to 20 carbon atoms, which may have a substituent" represented by $R^2$ to $R^{29}$ include the same as those of the "linear or branched alkyl group with 1 to 20 carbon atoms, which may have a substituent" represented by $R^1$ in the general formula (1).

In the general formula (1), examples of the "linear or branched alkenyl group with 2 to 20 carbon atoms" in the "linear or branched alkenyl group with 2 to 20 carbon atoms, which may have a substituent" represented by $R^2$ to $R^{29}$ include the same as those of the "linear or branched alkenyl group with 2 to 20 carbon atoms, which may have a substituent" represented by $R^1$ in the general formula (1).

In general formula (1), specifically, examples of the "cycloalkyl group with 3 to 12 carbon atoms" in the "cycloalkyl group with 3 to 12 carbon atoms, which may have a substituent" represented by $R^2$ to $R^{29}$ include a cyclopropyl group, a cyclobutyl group, a cyclopentyl group, a cyclohexyl group, a cycloheptyl group, a cyclooctyl group, a cyclodecyl group, a cyclododecyl group, a 4-methylcyclohexyl group, and a 4-ethylcyclohexyl group. The number of the carbon atoms in the cycloalkyl group may be selected in a range of 3 to 10, for example.

In the general formula (1), examples of the "linear or branched alkoxy group with 1 to 20 carbon atoms" in the "linear or branched alkoxy group with 1 to 20 carbon atoms, which may have a substituent" represented by $R^2$ to $R^{29}$ include the same as those of the "linear or branched alkoxy group with 1 to 20 carbon atoms, which may have a substituent" represented by $R^1$ in the general formula (1).

In the general formula (1), specifically, examples of the "amino group with 0 to 20 carbon atoms" in the "amino group with 0 to 20 carbon atoms, which may have a substituent" represented by $R^2$ to $R^{29}$ include the same as those of the "amino group with 0 to 20 carbon atoms among the "amino group with 0 to 60 carbon atoms, which may have a substituent" represented by $R^1$ in the general formula (1).

In the general formula (1), specifically, examples of the "thio group with 1 to 20 carbon atoms" in the "thio group with 1 to 20 carbon atoms, which may have a substituent" represented by $R^2$ to $R^{29}$ include the same as those of the "thio group with 1 to 20 carbon atoms, which may have a substituent" represented by $R^1$ in the general formula (1).

Examples of the "substituent" in the "linear or branched alkyl group with 1 to 20 carbon atoms, which may have a substituent", the "linear or branched alkenyl group with 2 to 20 carbon atoms, which may have a substituent", the "cycloalkyl group with 3 to 12 carbon atoms, which may have a substituent", the "linear or branched alkoxy group with 1 to 20 carbon atoms, which may have a substituent", the "amino group with 0 to 20 carbon atoms, which may have a substituent", or the "thio group with 0 to 20 carbon atoms, which may have a substituent" represented by $R^2$ to $R^{29}$ in the general formula (1) include the same as those of the "substituent" in the "linear or branched alkyl group with 1 to 20 carbon atoms, which may have a substituent" represented by $R^1$ in the general formula (1).

In the general formula (1), $R^{10}$ and $R^{11}$, and $R^{24}$ and $R^{25}$ each may bond to each other to form a ring. For example, $R^{10}$ and $R^{11}$, and $R^{24}$ and $R^{25}$ each may bond to each other via a single bond, an oxygen atom, a sulfur atom, a selenium atom or a nitrogen atom, thereby forming a ring. In one aspect of the invention, at least one pair of $R^{10}$ and $R^{11}$, and $R^{24}$ and $R^{25}$ bonds to each other via a single bond, and, for example, $R^{10}$ and $R^{11}$ may bond to each other via a single bond, and additionally $R^{24}$ and $R^{25}$ may bond to each other via a single bond. In one preferred aspect of the invention, neither $R^{10}$ and $R^{11}$ nor $R^{24}$ and $R^{25}$ bond to each other to form a ring. The compounds in which neither $R^{10}$ and $R^{11}$ nor $R^{24}$ and $R^{25}$ bond to each other to form a ring are preferred over the compounds in which they bond to each other to form a ring, because the former have higher solubility in solvents normally used.

In the general formula (1), preferably, at least one of $R^2$ to $R^{29}$ (preferably at least one of $R^6$ to $R^{15}$, and $R^{20}$ to $R^{29}$, more preferably at least one of $R^7$ to $R^9$, $R^{12}$ to $R^{14}$, $R^{21}$ to $R^{23}$, and $R^{26}$ to $R^{28}$) is a substituent that $R^2$ to $R^{29}$ can take. Of $R^2$

to $R^{29}$, the number of substituents preferably falls within a range of 1 to 12, and, for example, may be within a range of 1 to 8, or within a range of 4 to 8. When two or more of them are substituents, these substituents may be the same or different. Preferably, the groups are the same. In the invention, one or more selected from $R^8$, $R^{13}$, $R^{22}$ and $R^{27}$ is preferably a substituent, and for example, all of these may be substituents. In one aspect of the invention, examples of the substituent that $R^2$ to $R^{29}$ each can have include a linear or branched alkoxy group with 1 to 20 carbon atoms, which may have a substituent, a linear or branched alkylthio group with 1 to 20 carbon atoms, which may have a substituent, or an amino group (preferably a disubstituted amino group) with 0 to 60 carbon atoms, which may have a substituent. For example, the substituent that $R^2$ to $R^{29}$ can take is a linear or branched alkoxy group with 1 to 20 carbon atoms, which may have a substituent. In one aspect of the invention, $R^2$ to $R^5$, and $R^{16}$ to $R^{19}$ are hydrogen atoms. In one aspect of the invention, $R^6$, $R^{10}$, $R^{11}$, $R^{15}$, $R^{20}$, $R^{24}$, $R^{25}$ and $R^{29}$ are hydrogen atoms.

In the general formula (1), one triarylamino group bonds to any one of the 1- to 4-positions of the carbazole and one triarylamino group bonds to any one of the 5- to 8-positions of the carbazole. For example, one triarylamino group bonds to the 2- or 3-position of the carbazole, and one triarylamino group bonds to the 6- or 7-position of the carbazole. The bonding positions of the two carbazoles in the general formula (1) are preferably the same as each other. In the invention, preferably, the compound represented by the general formula (1) is a compound represented by the general formula (2) below, in which a triarylamino group is substituted at the 3- and 6-positions of the carbazole, or a compound represented by the general formula (3) below, in which a triarylamino group is substituted at the 2- and 7-positions of the carbazole. $R^2$ to $R^{29}$ in the general formula (2) and the general formula (3) have the same definition as $R^2$ to $R^{29}$ in the general formula (1) above.

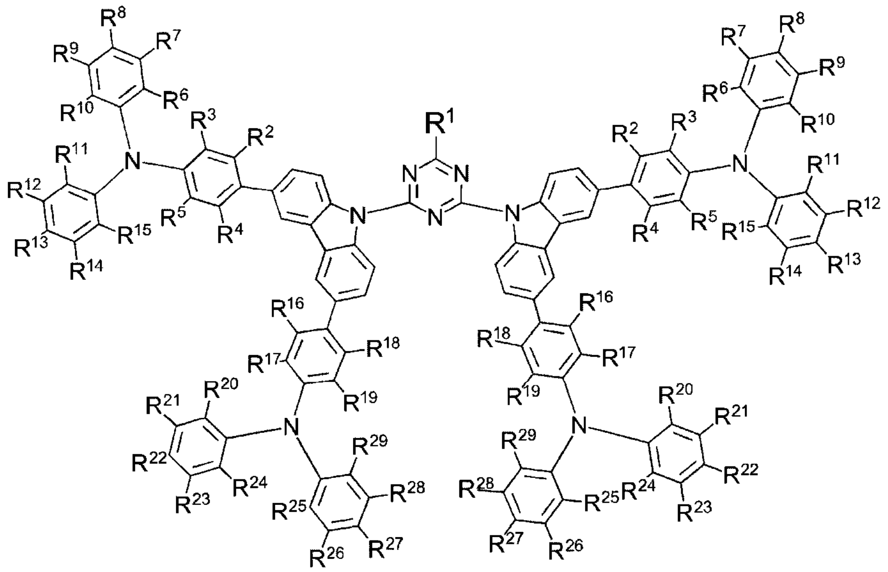

(2)

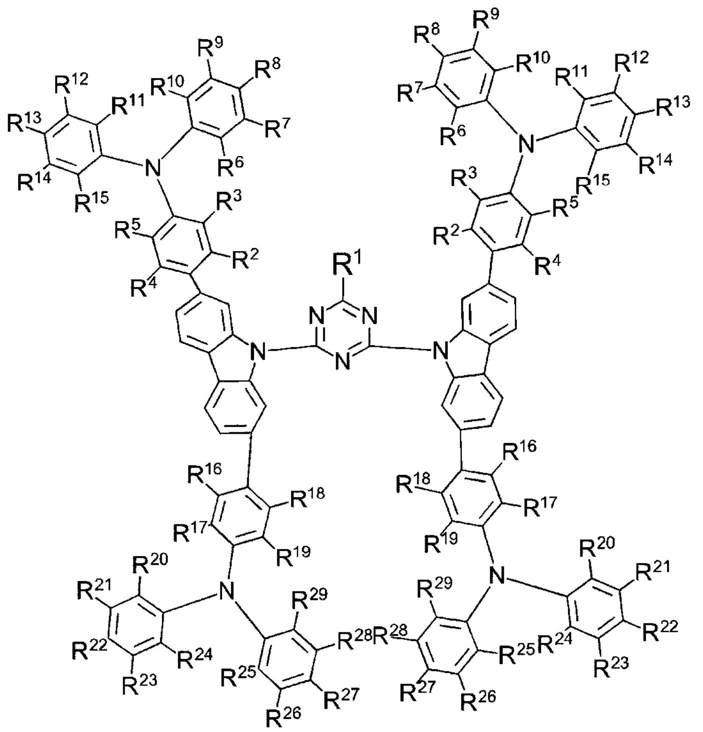

(3)

One group of compounds represented by the general formula (1) is Compound Group 1 in which $R^1$ is a monovalent heterocyclic group with 5 to 30 ring-forming atoms, which may have a substituent. Compound Group 1 includes Compound Group 1a in which $R^1$ is a monovalent aromatic heterocyclic group with 5 to 30 ring-forming atoms, which bonds via a carbon atom and which may have a substituent, Compound Group 1b in which $R^1$ is a diarylamino group with 5 to 30 ring-forming atoms (with the two aryl groups bonding to each other to form a cyclic structure), which may have a substituent, Compound Group 1c in which $R^1$ is a monovalent aromatic heterocyclic group with 5 to 30 ring-forming atoms, which bonds via a nitrogen atom and which may have a substituent (excepting those belonging to Compound Group 1b), Compound Group 1d in which $R^1$ is a monovalent aliphatic heterocyclic group with 5 to 30 ring-forming atoms, which bonds via a carbon atom and which may have a substituent, and Compound Group 1e in which $R^1$ is a monovalent aliphatic heterocyclic group with 5 to 30 ring-forming atoms, which bonds via a nitrogen atom and which may have a substituent. For example, in Compound Groups 1a to 1e, the heterocyclic group of each group has a substituent. For example, in Compound Groups 1a to 1e, the heterocyclic group of each group does not have a substituent, and is unsubstituted. Compound Groups 1a to 1e may each further satisfy at least one of the following additional requirements.

One additional requirement is that four to eight of $R^2$ to $R^{29}$ are substituents that $R^2$ to $R^{29}$ can take. One additional requirement is that at least one (e.g., all) of $R^8$, $R^{13}$, $R^{22}$, and $R^{27}$ is a substituent that $R^2$ to $R^{29}$ can take. One additional requirement is that at least one of $R^2$ to $R^{29}$ is a substituent, and the substituent that $R^2$ to $R^{29}$ can take is a linear or branched alkoxy group with 1 to 20 carbon atoms, which may have a substituent. One additional requirement is that at least one of $R^2$ to $R^{29}$ is a substituent, and the substituent that $R^2$ to $R^{29}$ can take is a linear or branched alkylthio group with 1 to 20 carbon atoms, which may have a substituent. One additional requirement is that at least one of $R^2$ to $R^{29}$ is a substituent, and the substituent that $R^2$ to $R^{29}$ take is a disubstituted amino group with 2 to 60 carbon atoms, which may have a substituent. One additional requirement is that the compound has the structure represented by the general formula (2) above. One additional requirement is that the compound has the structure represented by the general formula (3) above.

One group of compounds represented by the general formula (1) is Compound Group 2 in which $R^1$ is a monovalent aromatic hydrocarbon group with 6 to 30 carbon atoms, which may have a substituent. Compound Group 2 includes Compound Group 2a in which $R^1$ is an unsubstituted phenyl group, Compound Group 2b in which $R^1$ is a substituted phenyl group, Compound Group 2c in which $R^1$ is an unsubstituted fused aromatic hydrocarbon group, and Compound Group 2d in which $R^1$ is a fused aromatic hydrocarbon group having a substituent. For example, the substituent in Compound Groups 2b and 2d is selected from the above-mentioned Substituent Group A. For example, the substituent in Compound Groups 2b and 2d is an alkyl group or an aryl group. Compound Groups 2a to 2d each may further satisfy at least one additional requirement described for Compound Group 1.

One group of compounds represented by the general formula (1) is Compound Group 3 in which $R^1$ is an amino group with 0 to 60 carbon atoms, which may have a substituent. Compound Group 3 includes Compound Group 3a in which $R^1$ is an unsubstituted amino group, Compound Group 3b in which $R^1$ is a monosubstituted amino group, Compound Group 3c in which $R^1$ is a diarylamino group, which may have a substituent, and Compound Group 3d in which $R^1$ is a

dialkylamino group, which may have a substituent. For example, the substituent in Compound Groups 3b to 3d is selected from the above-mentioned Substituent Group A. For example, the substituent in Compound Groups 3b to 3d is an alkyl group or an aryl group. Compound Groups 3a to 3d each may further satisfy at least one additional requirement described for Compound Group 1.

One group of compounds represented by the general formula (1) is Compound Group 4 in which $R^1$ is a halogen atom. Compound Group 4 includes Compound Group 4a in which $R^1$ is a fluorine atom, Compound Group 4b in which $R^1$ is a chlorine atom, Compound Group 4c in which $R^1$ is a bromine atom, and Compound Group 4d in which $R^1$ is an iodine atom. Compound Groups 4a to 4d each may further satisfy at least one additional requirement described for Compound Group 1.

One group of compounds represented by the general formula (1) is Compound Group 5 in which $R^1$ is a linear or branched alkyl group with 1 to 20 carbon atoms, which may have a substituent.

One group of compounds represented by the general formula (1) is Compound Group 6 in which $R^1$ is a linear or branched alkenyl group with 2 to 20 carbon atoms, which may have a substituent.

One group of compounds represented by the general formula (1) is Compound Group 7 in which $R^1$ is a linear or branched alkoxy group with 1 to 20 carbon atoms, which may have a substituent.

One group of compounds represented by the general formula (1) is Compound Group 8 in which $R^1$ is an aryloxy group with 6 to 30 carbon atoms, which may have a substituent.

One group of compounds represented by the general formula (1) is Compound Group 9 in which $R^1$ is a thio group with 0 to 20 carbon atoms, which may have a substituent.

One group of compounds represented by the general formula (1) is Compound Group 10 in which $R^1$ is a hydrogen atom.

One group of compounds represented by the general formula (1) is Compound Group 11 in which $R^1$ is a hydroxyl group.

Compound Groups 5 to 11 each may further satisfy at least one additional requirement described for Compound Group 1.

Specific examples of the compound represented by the general formula (1) of the invention are shown below, but the compound represented by the general formula (1) which may be employed in the invention should not be interpreted in a limitative manner by these specific examples. In the following exemplary compounds, hydrogen atoms and carbon atoms are partly omitted, and some examples of existing isomers are exemplified here, therefore including any other all isomers. The compound may also be a mixture of two or more isomers thereof.

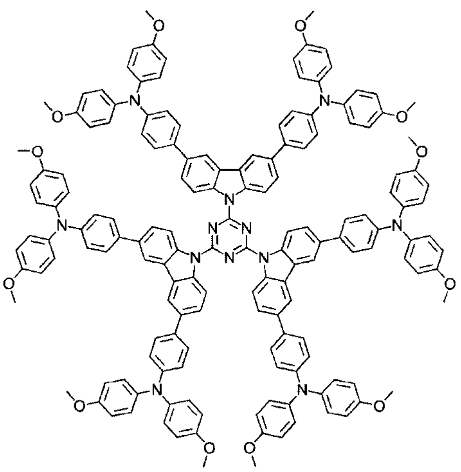

(A−1)

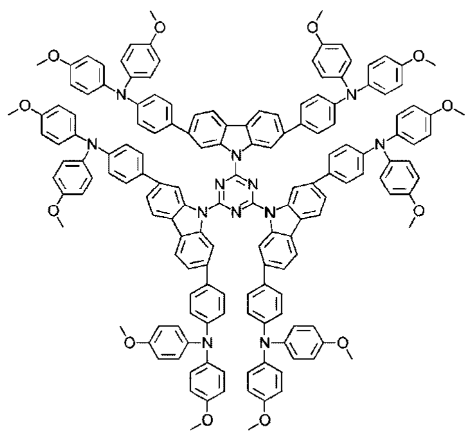

(A−2)

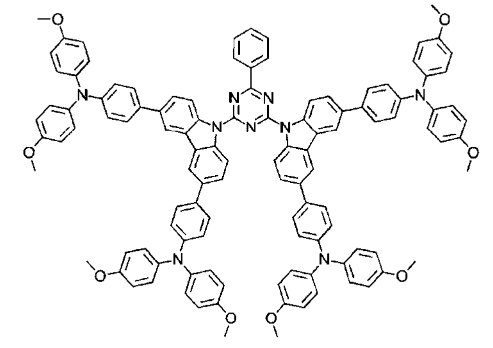

(A−3)

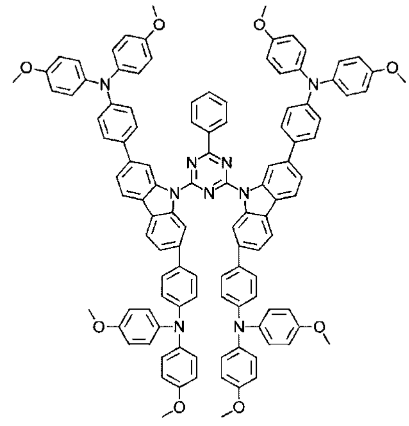

(A−4)

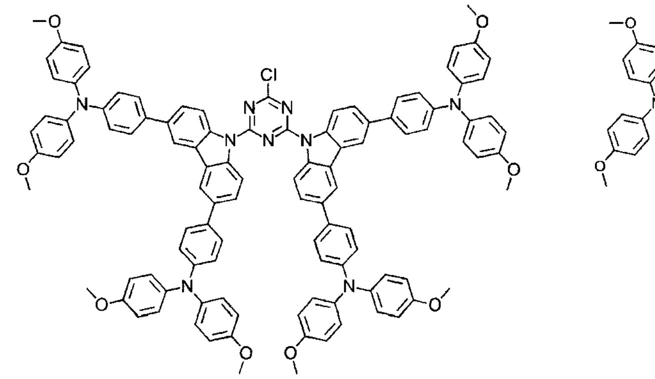

(A−5)

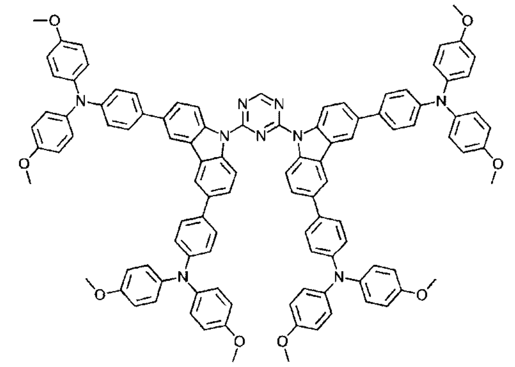

(A−6)

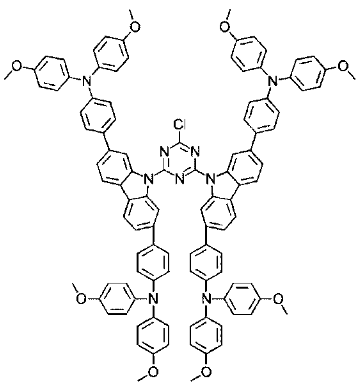

(A−7)

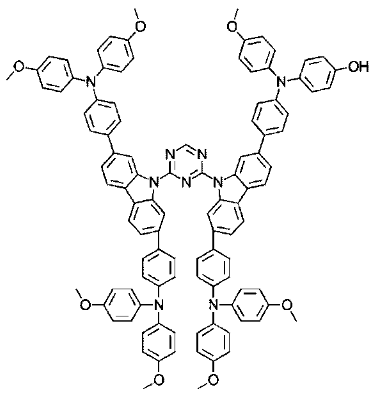

(A−8)

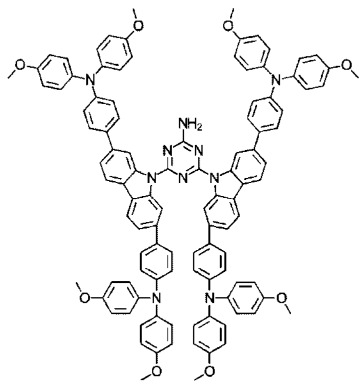

(A−9)

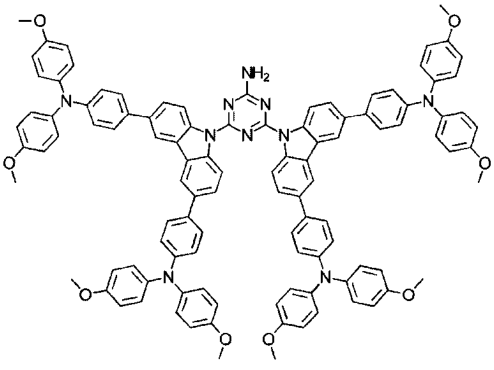

(A-10)

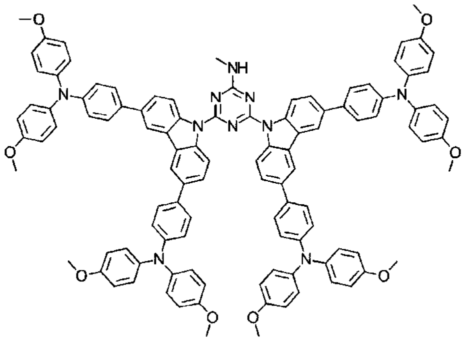

(A-11)

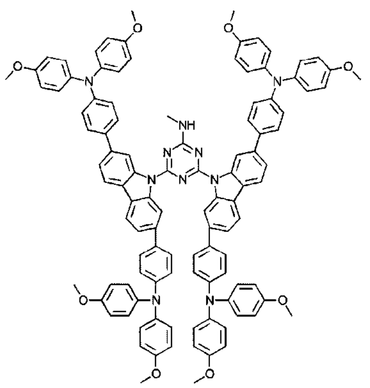

(A-12)

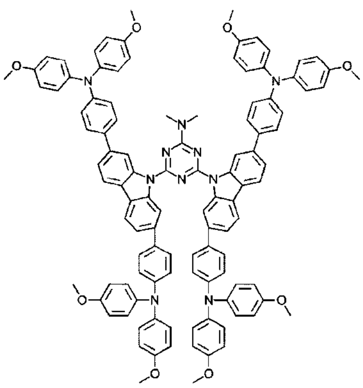

(A-13)

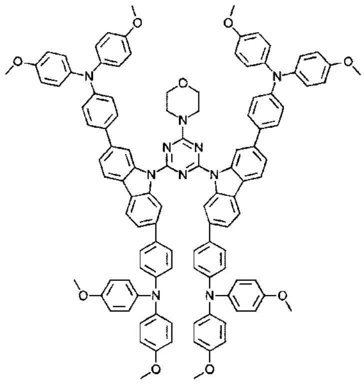

(A-14)

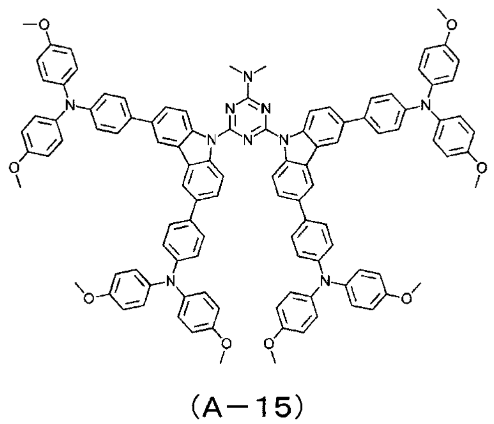

(A-15)

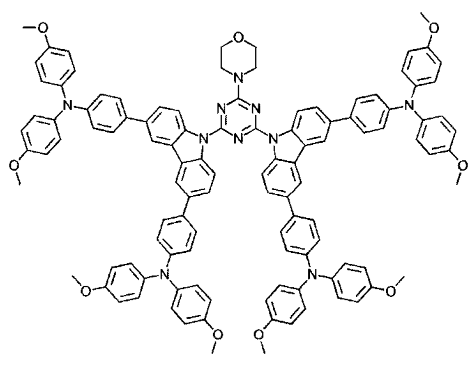

(A-16)

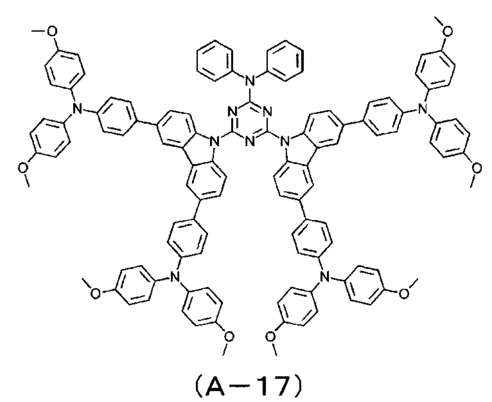

(A-17)

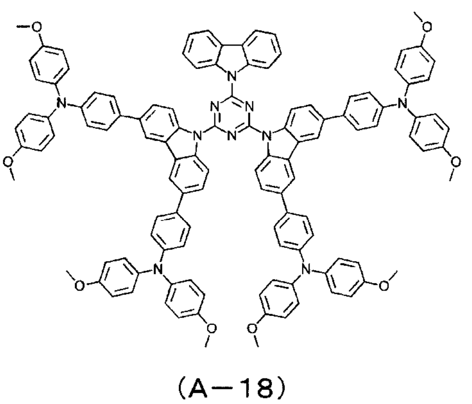

(A-18)

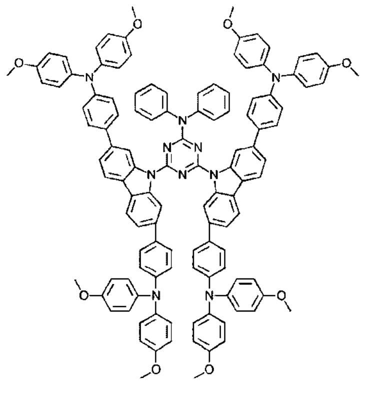

(A-19)

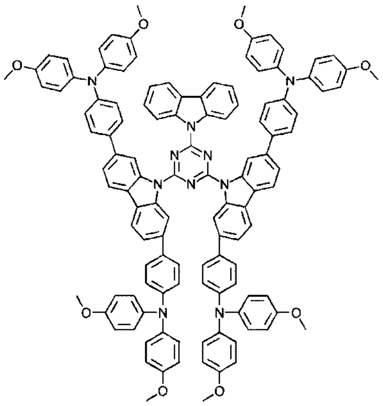

(A-20)

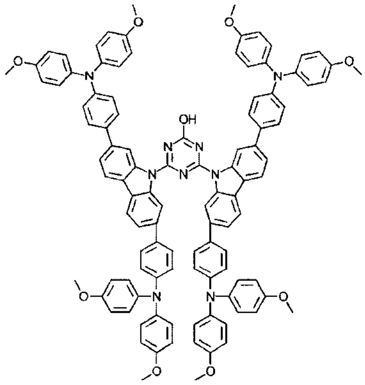

(A-21)

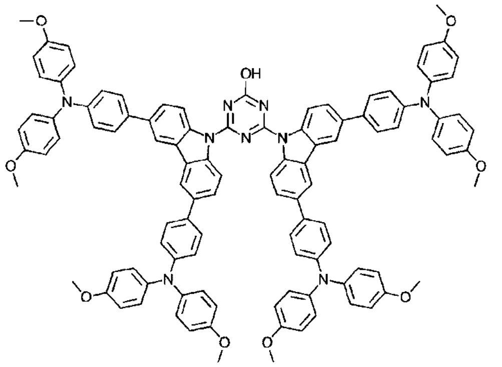

(A-22)

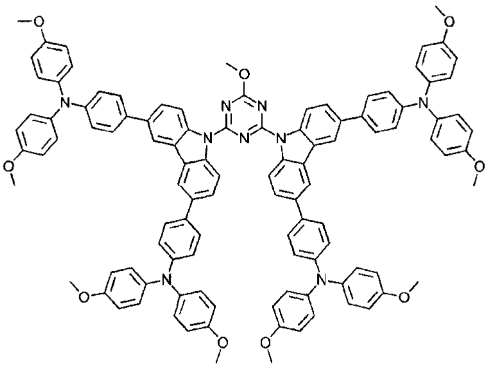

(A-23)

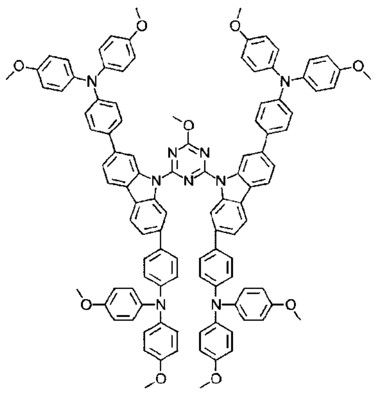

(A-24)

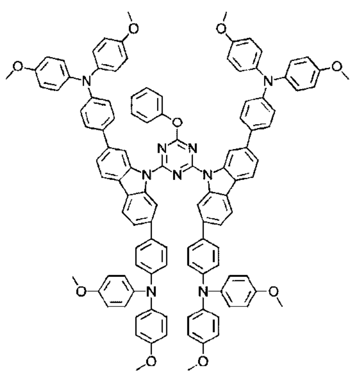

(A-25)

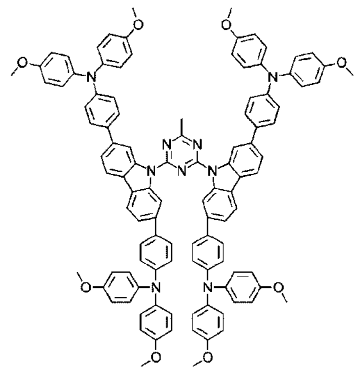

(A-26)

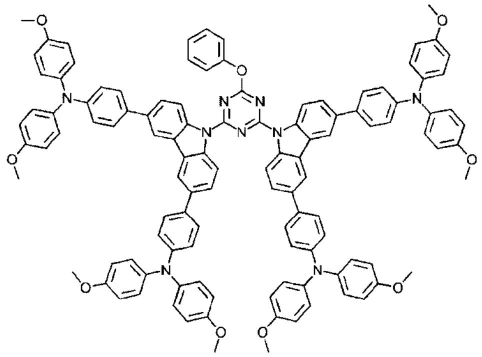

(A-27)

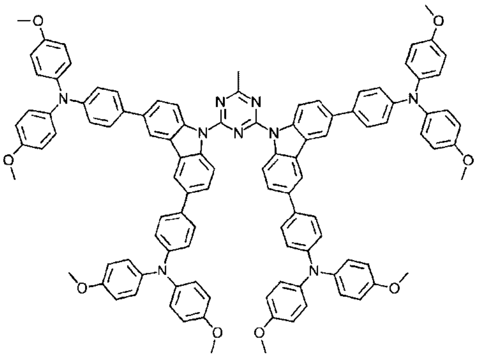

(A-28)

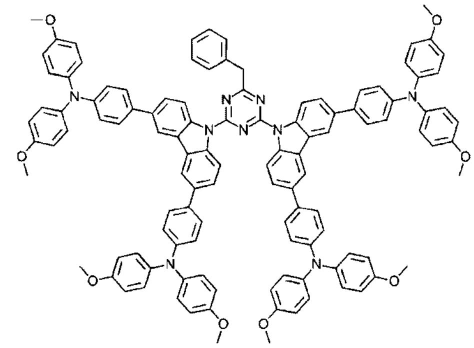

(A−29)

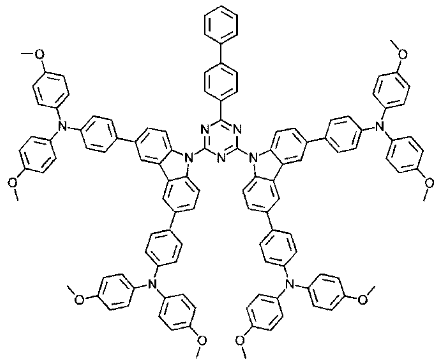

(A−30)

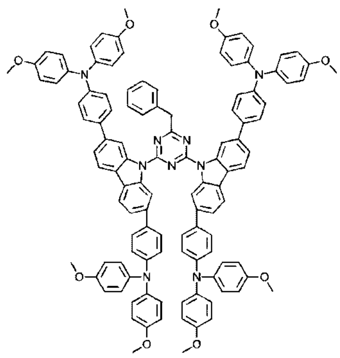

(A−31)

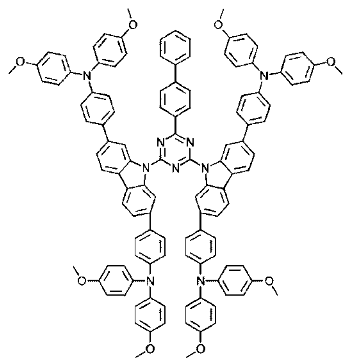

(A−32)

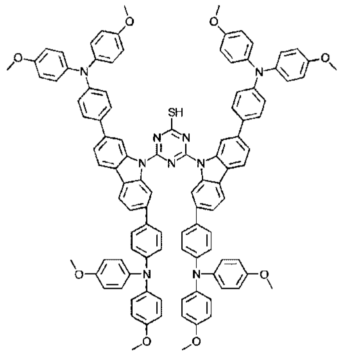

(A−33)

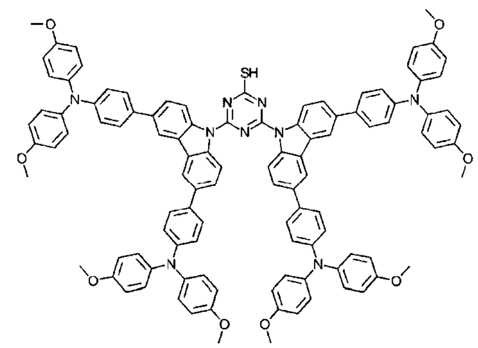

(A−34)

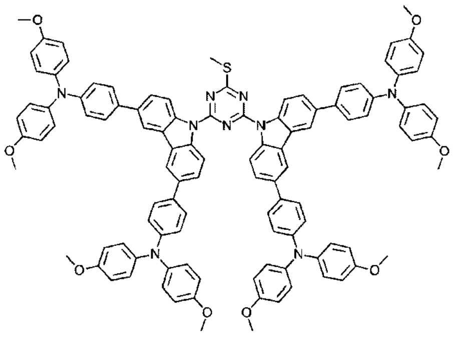

(A−35)

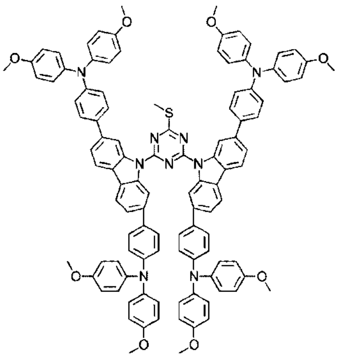

(A−36)

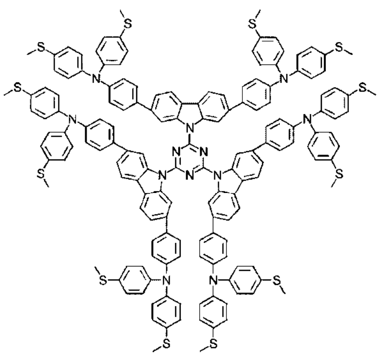

(A−37)

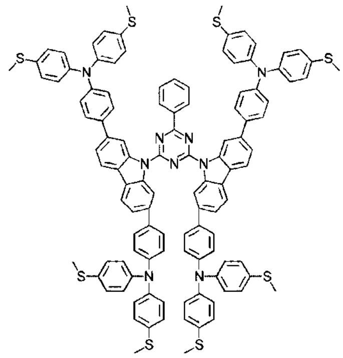

(A−38)

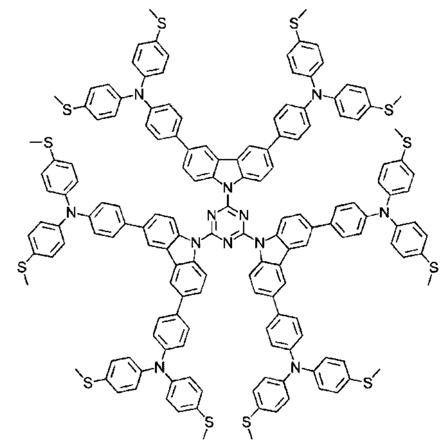

(A−39)

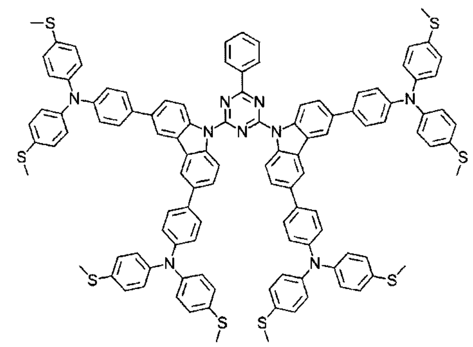

(A−40)

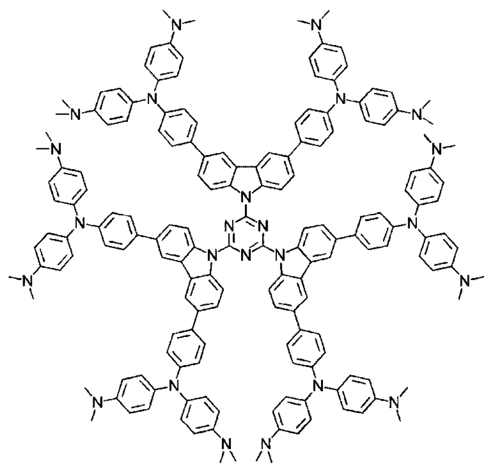

(A−41)

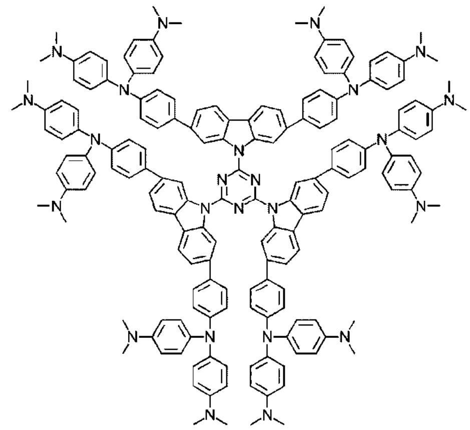

(A−42)

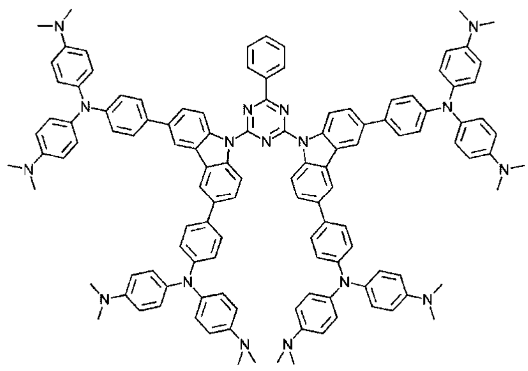

(A−43)

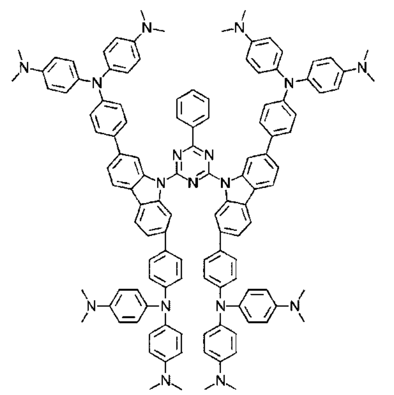

(A−44)

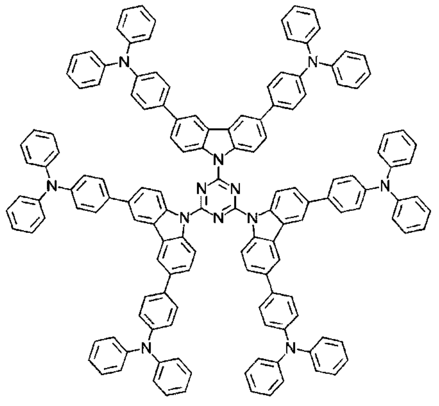

(A−45)

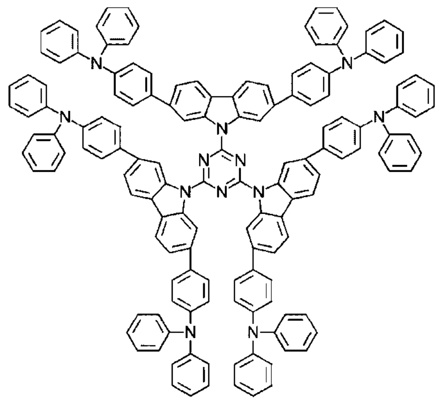

(A−46)

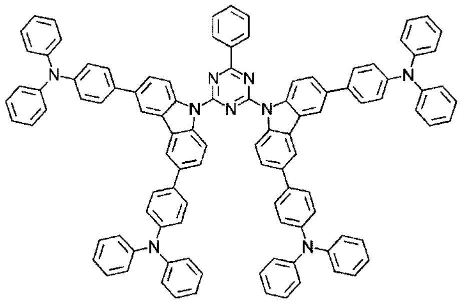

(A−47)

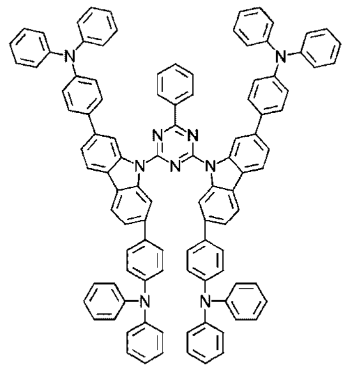

(A−48)

The compound represented by the general formula (1) of the invention can be synthesized using a known method. Of the compounds represented by the general formula (1), compounds represented by the general formula (2) and compounds represented by the general formula (3) can be synthesized, for example, by the methods described below.

By nucleophilic substitution reaction of a cyanuric acid chloride or a 2,4-dichloro-1,3,5-triazine derivative with a 3,6-triarylamino-substituted carbazole represented by the following general formula (4), the compounds of the above general formula (2) can be synthesized.

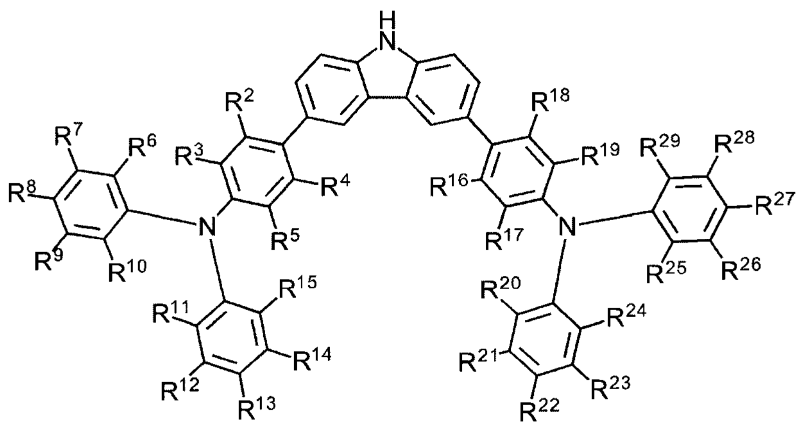

(4)

$R^2$ to $R^{29}$ in the above general formula (4) have the same definition as $R^2$ to $R^{29}$ in the general formula (1) above.

By nucleophilic substitution reaction of a cyanuric acid chloride or a 2,4-dichloro-1,3,5-triazine derivative with a 2,7-triarylamino-substituted carbazole represented by the following general formula (5), the compounds of the above general formula (3) can be synthesized.

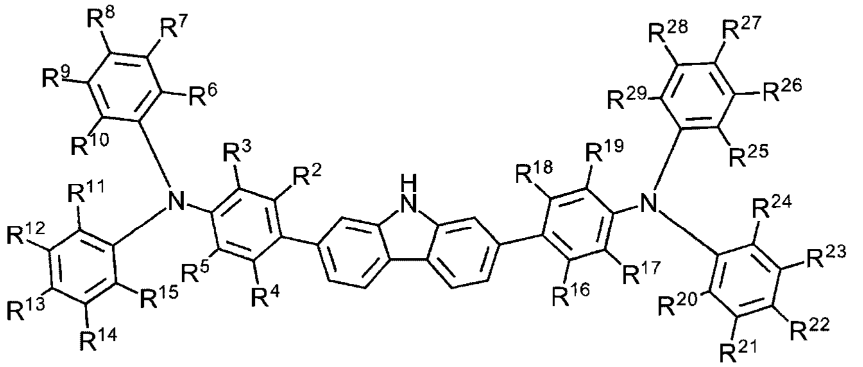

(5)

$R^2$ to $R^{29}$ in the above general formula (5) have the same definition as $R^2$ to $R^{29}$ in the general formula (1) above.

The compound represented by the general formula (1) of the invention may be purified by known methods, such as purification by column chromatography, adsorption purification using silica gel, activated charcoal, or activated clay, or solvent recrystallization, or crystallization. It may be effective to use a combination of these methods to increase the purity of the compound for use herein. These compounds can be identified by nuclear magnetic resonance analysis (NMR).

Preferred embodiments of the photoelectric conversion device of the invention are described below.

<Photoelectric Conversion Device>

Next, the photoelectric conversion device of the invention is described.

The photoelectric conversion device of the invention is characterized by having a hole transport layer that contains the compound represented by the general formula (1). For the description of the compound represented by the general formula (1), reference can be made to the description in the above section of <Compound Represented by General Formula (1)>. The compound represented by the general formula (1) can be effectively used as a material for the hole transport layer of photoelectric conversion devices because of its excellent hole transporting and electron blocking properties.

In the following, preferred embodiments of the photoelectric conversion device will be described, but embodiments of the photoelectric conversion device of the invention are not interpreted as limited by the embodiments shown below.

In one aspect of the invention, the photoelectric conversion device has, as shown in Fig. 1, a conductive support 1, an electron transport layer 2, a photoelectric conversion layer 3, a hole transport layer 4, and a counter electrode 5 in that order, and the hole transport layer 4 contains a compound represented by the general formula (1). In one aspect of the invention, the photoelectric conversion device has a conductive support, a hole transport layer, a photoelectric conversion layer, an electron transport layer, and a counter electrode in that order, and the hole transport layer contains a compound represented by the general formula (1). Here, the photoelectric conversion layer contains, for example, a perovskite-type compound. The photoelectric conversion device is, for example, a photoelectric conversion device used in solar cells.

In the following, the constituent members and layers of the photoelectric conversion device will be described using the photoelectric conversion device shown in Fig. 1 as an example.

[Conductive Support]

In the photoelectric conversion device shown in Fig. 1, the conductive support 1 functions as a cathode, taking out electrons transported from the photoelectric conversion layer 3 via the electron transport layer 2. In one aspect of the invention, the conductive support 1 is a conductive support having translucency that can transmit light to be used for photoelectric conversion, for example, a conductive substrate on which a film of conductive material is formed on a translucent substrate.

Specific examples of conductive material for use for the conductive support include conductive transparent oxide semiconductors such as tin-doped indium oxide (ITO), zinc-doped indium oxide (IZO), tungsten-doped indium oxide (IWO), zinc and aluminum oxides (AZO), fluorine-doped tin oxide (FTO), indium oxide ($In_2O_3$), and indium-tin composite oxide; and tin-doped indium oxide (ITO) and fluorine-doped tin oxide (FTO) are preferably used.

[Electron Transport Layer]

The electron transport layer 2 is a layer containing a material that has the function of transporting electrons (electron transport material), and is placed between the conductive support 1 and the photoelectric conversion layer 3 to

transport electrons generated in the photoelectric conversion layer 3 toward the conductive support 1. This improves the transfer efficiency of electrons from the photoelectric conversion layer to the conductive support. In addition to these functions, the electron transport layer may also have the function of suppressing hole injection from the conductive support. The electron transport layer 2 may be formed adjacent to the conductive support 1, or another layer may be interposed between the conductive support 1 and the electron transport layer 2.

Specific examples of the semiconductor material for use in the electron transport layer include metal oxides such as tin oxide (SnO, $SnO_2$, $SnO_3$, etc.), titanium oxide ($TiO_2$, etc.), tungsten oxide ($WO_2$, $WO_3$, $W_2O_3$, etc.), zinc oxide (ZnO), niobium oxide ($Nb_2O_5$, etc.), tantalum oxide ($Ta_2O_5$, etc.), yttrium oxide ($Y_2O_3$, etc.), and strontium titanate ($SrTiO_3$, etc.); metal sulfides such as titanium sulfide, zinc sulfide, zirconium sulfide, copper sulfide, tin sulfide, indium sulfide, tungsten sulfide, cadmium sulfide, and silver sulfide; metal selenides such as titanium selenide, zirconium selenide, indium selenide, and tungsten selenide; and elemental semiconductors such as silicon and germanium. One of these semiconductor materials may be used alone, or a combination of two or more of the semiconductor materials may be used. Preferred examples of semiconductor materials for the electron transport layer include one or a combination of two or more materials selected from tin oxide, titanium oxide, and zinc oxide.

Paste containing fine particles of the above semiconductor material (semiconductor paste) may be used as a material for forming the electron transport layer. The semiconductor paste may be a commercial product or a preparation made by dispersing fine powders of the above semiconductor material in a solvent. Specific examples of the solvent for use in the preparation of the semiconductor paste are: water; alcohol solvents such as methanol, ethanol, isopropyl alcohol; ketone solvents such as acetone, methyl ethyl ketone, methyl isobutyl ketone; and hydrocarbon solvents such as n-hexane, cyclohexane, benzene, and toluene, but these are not limitative. One of these solvents may be used alone, or a mixture of two or more of these solvents may be used as a mixed solvent.

Examples of the method of dispersing the semiconductor fine powder in a solvent include a method of mashing the powder in a mortar and pestle as needed and then dispersing it in a solvent using a dispersing machine such as a ball mill, a paint conditioner, a vertical bead mill, a horizontal bead mill, or an attritor. When preparing the paste, it is preferable to add a surfactant or the like to prevent the semiconductor particles from aggregating, and a thickener such as polyethylene glycol to thicken the paste.

The electron transport layer can be formed using a known film-forming method. In other words, the electron transport layer can be formed by a coating method using a coating liquid containing a semiconductor material (e.g., an electron transport layer-coating liquid such as semiconductor paste) or by a vapor phase process. Specifically, there can be mentioned a film-forming method in which a coating liquid for an electron transport layer is applied onto a conductive substrate according to a wet coating method such as a spin coating method, an inkjet method, a doctor blade method, a drop casting method, a squeegee method, a screen printing method, a reverse roll coating method, a gravure coating method, a kiss coating method, a roll brushing method, a spray coating method, an air knife coating method, a wire bar coating method, a pipe doctor method, an immersing coating method or a curtain coating method, and then the solvent and additives are removed by firing for film formation, as well as a method of forming a semiconductor material film according to a vapor phase film forming method such as a sputtering method, a vapor evaporation method, an electrodeposition method, an electrodialysis method or a microwave irradiation method. Among them, the coating method in which a prepared coating liquid for an electron transport layer is applied by spin-coating is preferred, but not limited to this. The spin-coating conditions can be set in any desired manner. The atmosphere in which the film is formed is not particularly limited and may be atmospheric or inert.

The thickness of the electron transport layer is, for example, 5 nm to 200 nm, preferably 10 nm to 150 nm. For example, when a dense electron transport layer is used from the viewpoint of further improving photoelectric conversion efficiency, the thickness of the electron transport layer is, in general, preferably 5 nm to 100 nm, more preferably 10 nm to 50 nm. In the invention, when a porous (mesoporous) metal oxide is used in addition to the dense layer, the thickness of the film is, in general, preferably 20 nm to 200 nm, more preferably 50 nm to 150 nm.

[Photoelectric Conversion Layer]

The photoelectric conversion layer 3 is a layer for converting light energy into electricity, more specifically, it is a layer that generates holes and electrons by creating a charge separation state by light energy. In the photoelectric conversion device shown in Fig. 1, the photoelectric conversion layer 3 is formed on the opposite side of the conductive support 1 from the electron transport layer 2.

An example of the photoelectric conversion layer is a layer formed of a perovskite material (perovskite layer). Here, the "perovskite material" means a material having a perovskite-type structure represented by a general formula $ABX_3$. In the general formula, A represents a monovalent organic cation or a monovalent metal cation, B represents a divalent metal cation, and X represents a halide ion. Examples of the monovalent cation represented by A include $K^+$, $Rb^+$, $Cs^+$, $CH_3NH_3^+$ (hereinunder MA: methylammonium), $NH=CHNH_2^+$ (hereinunder FA: formamidinium), and $CH_3CH_2NH_3^+$ (hereinunder EA: ethylammonium). Examples of the divalent metal cation represented by B include $Pb^{2+}$ and $Sn^{2+}$.

Examples of the halide ion represented by X include $I^-$ and $Br^-$.

Specific examples of the perovskite material include $MAPbI_3$, $FAPbI_3$, $EAPbI_3$, $CsPbI_3$, $MASnI_3$, $FASnI_3$, $EASnI_3$, $MAPbBr_3$, $FAPbBr_3$, $EAPbBr_3$, $MASnBr_3$, $FASnBr_3$, and $EASnBr_3$, and also include mixed cationic-type and mixed anionic-type perovskite materials such as $(FAMA)Pb(IBr)_3$, $K(FAMA)Pb(IBr)_3$, $Rb(FAMA)Pb(IBr)_3$, and $Cs(FAMA)Pb(IBr)_3$. The photoelectric conversion layer may contain only one selected from these perovskite materials or two or more selected from these perovskite materials.

The photoelectric conversion layer may consist solely of perovskite materials or may contain any other materials in addition to perovskite materials. The other materials include light absorbers.

The perovskite layer may be formed by applying a solution of a halide AX and a metal halide $BX_2$ (perovskite precursor solution) to form a precursor coating film and drying this precursor coating film. For specific examples of A, B, and X, reference can be made to the description of the ions constituting $ABX_3$ mentioned above. For example, specific examples of halides AX include methylammonium halides, formamidine halides and cesium halides, and specific examples of metal halides $BX_2$ include lead halides and tin halides.

From the standpoint of precursor solubility, examples of the solvent for the perovskite precursor solution include, but not limited to, N,N-dimethylformamide (DMF), dimethyl sulfoxide (DMSO), and γ-butyrolactone. One of these solvents may be used alone, or a mixture of two or more of these solvents may be used. A preferred example of the solvent is a mixed solvent of N,N-dimethylformamide and dimethyl sulfoxide. As the solvent, preferred is a dehydrated solvent with a moisture content of 10 ppm or less. Dehydration of the solvent may be performed using molecular sieves or the like.

The coating process with the perovskite precursor solution is performed preferably under a dry atmosphere, and it is more preferable to perform the coating process under a dry inert gas atmosphere such as a glove box. This prevents water from entering the perovskite layer, allowing high-efficiency perovskite solar cells to be manufactured with good reproducibility. For the coating method, the description of the coating method with the coating liquid for the electron transport layer described in the above [electron transport layer] section can be referred to.

The perovskite layer is formed by drying the precursor coating film thus formed. Drying of the precursor coating film may be done by natural drying or by heating drying using a hot plate or the like. The temperature at which the precursor coating film is heated with a hot plate or other means is preferably 50 to 200°C, and 70 to 150°C is more preferable from the viewpoint of forming the perovskite material from the precursor. The heating time is preferably 10 to 90 minutes, more preferably 10 to 60 minutes.

The thickness of the photoelectric conversion layer (perovskite layer) is preferably 50 to 1000 nm, more preferably 300 to 700 nm. This suppresses performance degradation due to defects or delamination of the photo conversion layer, avoids excessively high device resistance, and allows the photoelectric conversion layer to have sufficient light absorption.

[Hole Transport Layer]

In the photoelectric conversion device shown in Fig. 1, the hole transport layer 4 is a layer containing a material that has the function of transporting holes (hole transport material), and is placed between the photoelectric conversion layer 3 and the counter electrode 5, having the function of transporting the holes generated in the photoelectric conversion layer 3 toward the counter electrode 5. This improves the transfer efficiency of holes from the photoelectric conversion layer to the electrode. In addition to the function, the hole transport layer may also have the function of suppressing electron injection from the counter electrode.

In the photoelectric conversion device of the invention, the hole transport layer contains the compound represented by the general formula (1) as a hole transport material. The compound represented by the general formula (1), which the hole transport layer contains, may be one or two or more selected from the compound groups represented by the general formula (1). Containing the compound represented by the general formula (1), the hole transport layer may be a layer with high hole transport capacity and excellent function to block electron transfer from the counter electrode. In addition to the compound represented by general formula (1), the hole transport layer may also contain a hole transport material that does not correspond to the compound represented by general formula (1) (hereinafter referred to as "second hole transport material") and additives.

The second hole transport material may be an inorganic hole transport material or an organic hole transport material. Specific examples of the inorganic hole transport material include compound semiconductors containing a monovalent copper, such as CuI, $CuInSe_2$, CuS; compounds containing any other metal than copper, such as GaP, NiO, CoO, FeO, $Bi_2O_3$, $MoO_2$, $Cr_2O_3$. Examples of the organic hole transport material include polythiophene derivatives such as poly-3-hexylthiophene (P3HT) and polyethylene dioxythiophene (PEDOT); fluorene derivatives such as 2,2',7,7'-tetrakis-(N,N-di-p-methoxyphenylamine)-9,9'-spirobifluorene (Spiro-OMeTAD); carbazole derivatives such as polyvinylcarbazole; triphenylamine derivatives such as poly[bis(4-phenyl)(2,4,6-triphenyl)amine] (PTAA); diphenylamine derivatives; polysilane derivatives; and polyaniline derivatives. These second hole transport materials may be mixed in the hole transport layer, or a hole transport layer containing the second hole transport material may be stacked on top of the hole

transport layer containing the compound represented by the general formula (1). In one aspect of the invention, the hole transport layer is a single layer and contains only the compound represented by the general formula (1) as the hole transport material.

For the method of forming the hole transport layer, reference can be made to the description relating to the method of forming the electron transport layer mentioned above. The following may be used as solvents for the coating liquid for the hole transport layer.

Specifically, examples of the solvent usable for the coating liquid for the hole transport layer include aromatic organic solvents such as benzene, toluene, xylene, mesitylene, tetralin (1,2,3,4-tetrahydronaphthalene), monochlorobenzene (chlorobenzene), o-dichlorobenzene, m-dichlorobenzene, p-dichlorobenzene and nitrobenzene; alkyl halide organic solvents such as dichloromethane, chloroform, 1,2-dichloroethane, 1,1,2-trichloroethane, dichloromethane; nitrile solvents such as benzonitrile and acetonitrile; ether solvents such as tetrahydrofuran, dioxane, diisopropyl ether, c-pentyl methyl ether, ethylene glycol dimethyl ether, ethylene glycol diethyl ether, and propylene glycol monomethyl ether; ester solvents such as ethyl acetate, and propylene glycol monomethyl ether acetate; and alcohol solvents such as methanol, isopropanol, n-butanol, propylene glycol, 1,3-butanediol, 1,4-butanediol, 2,3-butanediol, cyclohexanol, and 2-n-butoxyethanol, but are not limited to these solvents. One of these solvents may be used alone, or a mixture of two or more of these solvents may be used. Above all, aromatic organic solvents and alkyl halide organic solvents are preferred as the solvent for the coating liquid for forming the hole transport layer.

The atmosphere during film formation for the hole transport layer is preferably a dry atmosphere. As the solvent for the coating liquid, preferred is a dehydrated solvent with a moisture content of 10 ppm or less. Highly efficient perovskite solar cells can be produced with good reproducibility by preventing water contamination.

From the viewpoint of improving the photoelectric conversion efficiency, the thickness of the hole transport layer is preferably 5 nm to 500 nm, more preferably 10 nm to 250 nm.

[Additive]

Examples of additives that may be added to the hole transport layer include oxidants (dopants) and basic compounds (basic additives). Adding these additives to the hole transport layer improves the carrier concentration in the hole transport layer, thereby increasing the photoelectric conversion efficiency of the photoelectric conversion device.

Specific examples of the dopant include bis(trifluoromethylsulfonyl)imidolithium (LiTFSI), bis(trifluoromethanesulfonyl)imide silver, bis(trifluoromethanesulfonyl)imide zinc(II), bis(trifluoromethanesulfonyl)imide copper(II), bis(trifluoromethanesulfonyl)imide magnesium(II), bis(trifluoromethanesulfonyl)imide calcium(II), tris(2-(1H-pyrazol-1-yl)-4-tert-butylpyridine)cobalt(III) tri[bis(trifluoromethane)sulfonimide] (FK209), $NOSbF_6$, $SbCl_5$, $SbF_5$; and above all, use of bis(trifluoromethylsulfonyl)imidolithium (LiTFSI) is preferred.

The concentration of the dopant in the hole transport layer is preferably 2.0 equivalents or less per equivalent of the hole transport material, more preferably 0.5 equivalents or less. While the inclusion of additives in the hole transport layer improves the photoelectric conversion efficiency of the photoelectric conversion device, too high a concentration of the dopant may reduce the durability of the photoelectric conversion device.

Specific examples of the basic additive include 4-tert-butylpyridine (tBP), 2-picoline, and 2,6-rutidine; and above all, 4-tert-butylpyridine is preferred. The basic additive may be used in combination with the dopant.

The concentration of the basic additive in the hole transport layer is preferably 5 equivalents or less per equivalent of the hole transport material, more preferably 3.5 equivalents or less.

[Counter Electrode]

The counter electrode 5 is an electrode formed on the opposite side of the hole transport layer 4 to the photoelectric conversion layer 3, and is positioned opposite to the conductive support 1 with the above electron transport layer 2, the photoelectric conversion layer 3 and the hole transport layer 4 therebetween. The counter electrode functions as an anode, taking out holes transported from the photoelectric conversion layer via the hole transport layer. The counter electrode 5 may be provided adjacent to the hole transport layer 4, or an electron blocking layer made of an organic material or inorganic compound semiconductor may be interposed between the hole transport layer 4 and the counter electrode 5.

The counter electrode is specifically made of metals such as platinum, titanium, stainless steel, aluminum, gold, silver, nickel, magnesium, chromium, cobalt, or copper, or alloys thereof. Among these, gold, silver, or silver alloys are preferred because they exhibit high electrical conductivity in thin films. Silver alloys are less susceptible to sulfidation or chlorination and more stable as thin films, and examples of silver alloys include silver-gold alloys, silver-copper alloys, silver-palladium alloys, silver-copper-palladium alloys, and silver-platinum alloys. The counter electrode is preferably a material that may be formed in a vapor phase process such as vapor deposition.

When a metal electrode is used as the counter electrode, its film thickness is preferably 10 nm or more to attain

good conductivity, more preferably 50 nm or more.

In the photoelectric conversion device shown in Fig. 1, the conductive support 1 serves as a cathode and the counter electrode 5 serves as an anode. Preferably, the device is irradiated with light such as sunlight (light for photoelectric conversion) from the conductive support side. Upon irradiation with sunlight or the like, the photoelectric conversion layer absorbs light to be in an excited state, thereby producing electrons and holes. The electrons move through the electron transport layer to the conductive support, and the holes move through the hole transport layer to the counter electrode, resulting in the flow of electric current and functioning as a photoelectric conversion device.

The photoelectric conversion device of the invention may also have a conductive support, a hole transport layer, a photoelectric conversion layer, an electron transport layer, and a counter electrode, in that order. In that case, the conductive support functions as an anode and the counter electrode as a cathode, and the electrons generated in the photoelectric conversion layer move to the counter electrode via the electron transport layer, and the holes generated in the photoelectric conversion layer move to the conductive support via the hole transport layer. This allows the current to be extracted to the outside. For the description and specific examples of the materials of the constituent parts and layers used in this aspect, reference can be made to the corresponding description of the photoelectric conversion device previously shown in Fig. 1.

For evaluating the performance (characteristics) of the photoelectric conversion device of the invention, the short-circuit current density, the open circuit voltage, the fill factor, and the photoelectric conversion efficiency are measured. The short-circuit current density is a current per $cm^2$ flowing between the two terminals when the output terminals are shorted, and the open-circuit voltage is a voltage between the two terminals when the output terminals are opened. The fill factor is a value calculated by dividing the maximum output (product of current and voltage) by the product of the short-circuit current density and the open-circuit voltage, and depends mainly on the internal resistance. The photoelectric conversion efficiency is calculated by dividing the maximum output power (W) by the light intensity per square centimeter ($W/cm^2$) and then multiplying the result by 100 to express it as a percentage. The initial photoelectric conversion efficiency of the photoelectric conversion device having the device configuration of the invention can be determined to be good when it shows 10% or more.

The photoelectric conversion device of the invention can be applied to solar cells and various types of optical sensors. Solar cells to which the photoelectric conversion device of the invention is applied are preferably perovskite solar cells. Solar cells may be fabricated by preparing a necessary number of photoelectric conversion devices each containing the compound represented by the general formula (1) in the hole transport layer as cells, arranging the cells into a module, and providing predetermined electrical wiring.

## EXAMPLES

In the following, Examples are given to specifically describe the characteristics of the invention. The materials, processes and procedures shown below can be appropriately modified unless they deviate from the gist of the invention. Therefore, the scope of the invention is not limited to the following Examples. The identification of the compounds obtained in the synthesis examples was performed by $^1$H-NMR ($^1$H-NMR (nuclear magnetic resonance system by JEOL Ltd., JNM-ECZ400S/L1 Model).

### (Synthesis Example 1) Synthesis of Compound (A-1)

In a reaction vessel, 3,6-bis(4-(bis(4-methoxyphenyl)amino)phenyl)-9H-carbazole (336 mg, Tokyo Chemical Co.), DMF (10 m L), and sodium hydride (19 mg, purity 55%, Kanto Chemical Co., Inc.) were put, and stirred in an ice bath for 30 minutes. Further, cyanuric acid chloride (25 mg, Tokyo Chemical Industry Co., Ltd.) was added, and the mixture was stirred for 1 hour at room temperature. After the reaction was completed, water (20 mL) was added and the precipitated solid was collected by suction filtration. The filtered crude product was purified by silica gel column (toluene:ethyl acetate = 20:1) to give compound (A-1) as a light yellow powder (yield: 263 mg, yield: 81%).

$^1$H-NMR(400MHz, DMSO-$d_6$): $\delta$(ppm) = 8.71(6H), 8.34(6H), 7.48(12H), 7.38(6H), 6.95(24H), 6.83(24H), 3.71(36H)

### [Synthesis Example 2] Synthesis of Compound (A-2)

In a reaction vessel, 2,7-bis(4-(bis(4-methoxyphenyl)amino)phenyl)-9H-carbazole (168 mg, Tokyo Chemical Co.), DMF (3 m L), and sodium hydride (10 mg, purity 55%, Kanto Chemical Co., Inc.) were put, and stirred in an ice bath for 30 minutes. Further, cyanuric acid chloride (15 mg, Tokyo Chemical Industry Co., Ltd.) was added, and the mixture was stirred for 1 hour at room temperature. After the reaction was completed, water (20 mL) was added and the precipitated solid was collected by suction filtration. The filtered crude product was purified by silica gel column (toluene:ethyl acetate = 20:1) to give compound (A-2) as a light yellow powder (yield: 74 mg, yield: 47%).

$^1$H-NMR(400MHz, DMSO-$d_6$): $\delta$(ppm) = 9.15(6H), 8.13(6H), 7.58(6H), 7.13(12H), 6.84(48H), 3.72(36H)

[Synthesis Example 3] Synthesis of Compound (A-3)

In a reaction vessel, 3,6-bis(4-(bis(4-methoxyphenyl)amino)phenyl)-9H-carbazole (143 mg, Tokyo Chemical Co.), DMF (3 m L), and sodium hydride (9 mg, purity 55%, Kanto Chemical Co., Inc.) were put, and stirred in an ice bath for 30 minutes. Further, 2,4-dichloro-6-phenyl-1,3,5-triazine (20 mg, Tokyo Chemical Industry Co., Ltd.) was added, and the mixture was stirred for 30 minutes at room temperature. After the reaction was completed, water (20 mL) was added and the precipitated solid was collected by suction filtration. The filtered crude product was purified by silica gel column (toluene:ethyl acetate = 6:1) to give compound (A-3) as a yellow powder (yield: 104 mg, yield: 69%).
$^1$H-NMR(400MHz, DMSO-$d_6$): δ(ppm) = 8.82(4H), 8.58(2H), 8.46(4H), 7.68(3H), 7.63(4H), 7.55(8H), 6.95(16H), 6.84(16H), 6.78(8H), 3.69(24H)

[Synthesis Example 4] Synthesis of Compound (A-4)

In a reaction vessel, 2,7-bis(4-(bis(4-methoxyphenyl)amino)phenyl)-9H-carbazole (143 mg, Tokyo Chemical Co.), DMF (3 m L), and sodium hydride (10 mg, purity 55%, Kanto Chemical Co., Inc.) were put, and stirred in an ice bath for 30 minutes. Further, 2,4-dichloro-6-phenyl-1,3,5-triazine (20 mg, Tokyo Chemical Industry Co., Ltd.) was added, and the mixture was stirred for 1.5 hours at room temperature. After the reaction was completed, water (20 mL) was added and the precipitated solid was collected by suction filtration. The filtered crude product was washed with methanol and ethyl acetate to give Compound (A-4) as a yellow powder (yield: 96 mg, yield: 64%).
$^1$H-NMR(400MHz, DMSO-$d_6$): δ(ppm) = 9.15(4H), 8.61(2H), 8.05(4H), 7.65(1H), 7.57(4H), 7.49(2H), 7.34(8H), 6.93(16H), 6.85(16H), 6.65(8H), 3.72(24H)

[Synthesis Example 5] Synthesis of Compound (A-5)

In a reaction vessel, 3,6-bis(4-(bis(4-methoxyphenyl)amino)phenyl)-9H-carbazole (600 mg, Tokyo Chemical Co.), DMF (12 m L), and sodium hydride (34 mg, Kanto Chemical Co., Inc.) were put, and stirred in an ice bath for 30 minutes. The reaction system was cooled down to -10°C, then cyanuric acid chloride (23 mg, Tokyo Chemical Industry Co., Ltd.) was added, and the mixture was stirred at -10°C for 2 hours and then at 0°C for 90minutes. After the reaction was completed, water (50 mL) was added and the precipitated solid was collected by suction filtration. The filtered crude product was purified by silica gel column (toluene:ethyl acetate = 50:1 → 30:1 → 10:1) to give compound (A-5) as a yellow powder (yield: 200 mg, yield: 30%).
$^1$H-NMR(400MHz, DMSO-$d_6$): δ(ppm) = 8.53(4H), 8.22(4H), 7.46(12H), 6.77-6.93(40H), 3.72(24H)

[Synthesis Example 6] Synthesis of Compound (A-16)

In a reaction vessel, 3,6-bis(4-(bis(4-methoxyphenyl)amino)phenyl)-9H-carbazole (280 mg, Tokyo Chemical Co.), DMF (5 m L), and sodium hydride (16 mg, Kanto Chemical Co., Inc.) were put, and stirred in an ice bath for 30 minutes. Further, 2,4-dichloro-6-morpholino-1,3,5-triazine (40 mg, Tokyo Chemical Industry Co., Ltd.) was added, and the mixture was stirred for 90 minutes at room temperature. After the reaction was completed, water (50 mL) was added and the precipitated solid was collected by suction filtration. The filtered crude product was purified by silica gel column (toluene:ethyl acetate = 10:1) to give compound (A-16) as a light yellow powder (yield: 99 mg, yield: 33%).
$^1$H-NMR(400MHz, DMSO-$d_6$): δ(ppm) = 8.65(4H), 8.47(4H), 7.53(12H), 6.94(16H), 6.84(16H), 6.76(8H), 3.99(4H), 3.81-3.87(4H), 3.70(24H)

[Example 1] Preparation of Photoelectric Conversion Device using Compound (A-1)

Glass with an ITO film (conductive support 1, GEOMATEC Co., Ltd.) was ultrasonically cleaned with isopropyl alcohol and treated with UV ozone. Then, the following electron transport layer 2, photoelectric conversion layer 3, and hole transport layer 4 were formed by a coating method under a dry atmosphere having a relative humidity of 10% or less.
A tin oxide colloidal solution (tin oxide dispersion of tin(IV) oxide, 15% in $H_2O$ colloidal dispersion (Alfa Aesar) mixed with purified water at 1:1 (volume ratio) (coating liquid for electron transport layer) was spin-coated on the ITO film. Subsequently, this was heated on a hot plate at 150°C for 30 minutes to form a tin oxide layer (electron transport layer 2) with a thickness of about 20 nm.
Formamidine hydroiodide (1M, Tokyo Chemical Industry Co., Ltd.), lead(II) iodide (1.1M, Tokyo Chemical Industry Co., Ltd.), methylamine hydrobromide (0.2M, Tokyo Chemical Industry Co., Ltd.), and lead(II) bromide (0.2M, Tokyo Chemical Industry Co., Ltd.) were dissolved in a mixed solvent of dimethylformamide:dimethylsulfoxide = 4:1 (volume ratio), and further a dimethyl sulfoxide solution of cesium iodide (1.5 M, Tokyo Chemical Industry Co., Ltd.) was added to prepare a perovskite precursor solution. Here, the cesium iodide solution was added in such an amount that the

amount of cesium could be 5% of the composition.

In a dry atmosphere having a relative humidity of 10% RH, the prepared perovskite precursor solution was dropped onto the tin oxide layer and spin-coated with dropwise applying chlorobenzene (0.35 mL) thereto to form a perovskite precursor coating film. Subsequently, this was heated on a hot plate at 100°C for 1 hour to form a perovskite layer (photoelectric conversion layer 3) of $Cs(MAFA)Pb(IBr)_3$ with a thickness of about 500 nm.

As a dopant, bis(trifluoromethanesulfonyl)imidolithium was dissolved in acetonitrile at a concentration of 1.8 M to be a dopant solution. Compound (A-1), the hole transport material obtained in Synthesis Example 1, was dissolved in chlorobenzene at 70°C at a concentration of 30 mM. 4-Tert-butylpyridine was added thereto in an amount equal to 3.3 equivalents of Compound (A-1). Further, the dopant solution was added so that bis(trifluoromethanesulfonyl)imidolithium therein could be 0.5 equivalents relative to compound (A-1) to prepare a coating solution for hole transport layer.

The coating solution for hole transport layer was spin-coated onto the $Cs(MAFA)Pb(IBr)_3$ layer (photoelectric conversion layer 3) to form a hole transport layer 4 with a thickness of about 200 nm.

On the top of the hole transport layer, a gold electrode (counter electrode 5) was formed by depositing about 80 nm of gold in a vacuum evaporation method at a vacuum degree of about $1 \times 10^{-4}$ Pa to fabricate a photoelectric conversion device.

[Example 2] Preparation of Photoelectric Conversion Device using Compound (A-3)

A photoelectric conversion device was prepared in the same way as in Example 1, except that Compound (A-3) was dissolved in chlorobenzene at a concentration of 50 mM at room temperature instead of Compound (A-1), and spin-coated.

[Example 3] Preparation of Photoelectric Conversion Device using Compound (A-4)

A photoelectric conversion device was prepared in the same way as in Example 1, except that Compound (A-4) was dissolved in chlorobenzene at a concentration of 50 mM at room temperature instead of Compound (A-1), and spin-coated.

[Example 4] Preparation of Photoelectric Conversion Device using Compound (A-5)

A photoelectric conversion device was prepared in the same way as in Example 1, except that Compound (A-5) was dissolved in chlorobenzene at a concentration of 27 mM at room temperature instead of Compound (A-1), and spin-coated.

[Example 5] Preparation of Photoelectric Conversion Device using Compound (A-16)

A photoelectric conversion device was prepared in the same way as in Example 1, except that Compound (A-16) was dissolved in chlorobenzene at a concentration of 26 mM at room temperature instead of Compound (A-1), and spin-coated.

[Comparative Example 1] Preparation of Photoelectric Conversion Device using Comparative Compound (B-1)

A photoelectric conversion device was prepared in the same way as in Example 1, except that a standard hole transport material represented by the following formula (B-1), Spiro-OMeTAD (Sigma-Aldrich) was dissolved in chlorobenzene at a concentration of 70 mM at room temperature and used, instead of Compound (A-1).

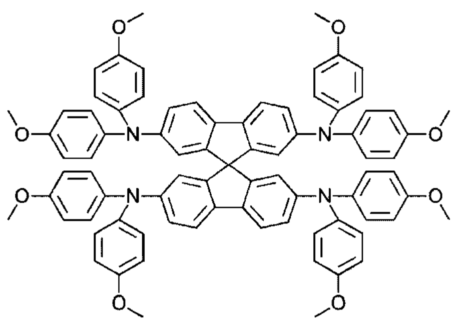

(B－1)

[Characteristic Evaluation]

The photoelectric conversion devices fabricated in Examples 1 to 5, and Comparative Example 1 each were irradiated with pseudo-sunlight (AM 1.5, 1000 $W/m^2$ ) generated by a white light irradiation device (Bunkoukeiki Co., Ltd., OTENTO-SUN SH model) from the conductive support side of the photoelectric conversion device, and the current-voltage characteristics thereof were measured with a source meter (Keithley Instruments, Inc., Model 2400 Series Source Meter) to provide the initial photoelectric conversion efficiency of each device.

After measurement of the current-voltage characteristics, each photoelectric conversion device was sealed in a laminated bag with a zipper (AL-8, SEISANNIPPONSHA LTD.) in a glove box in a nitrogen atmosphere. The encapsulated photoelectric conversion devices each were put into a vacuum constant-temperature dryer (VOS-310C, TOKYO RIKAKIKAI CO., LTD.), and stored at 85°C for 1,000 hours; then the devices were again irradiated with pseudo-sunlight and the current-voltage characteristics thereof were measured; and the photoelectric conversion efficiency of each device heated for 1,000 hours was determined.

Table 1 shows the retention rate (%) calculated from the following equation (a-1) using the resultant data of the initial photoelectric conversion efficiency and the photoelectric conversion efficiency after heating for 1,000 hours.

$$Retention\ Rate(\%) = \frac{Photoelectric\ Conversion\ Efficiency\ after\ heating\ for\ 1{,}000\ hours}{Initial\ Photoelectric\ Conversion\ Efficiency} \times 100 \quad \text{(a-1)}$$

[Table 1]

| | Hole Transport Material | Retention Rate [%] |
|---|---|---|
| Example 1 | A-1 | 94.95 |
| Example 2 | A-3 | 66.19 |
| Example 3 | A-4 | 99.58 |
| Example 4 | A-5 | 66.34 |
| Example 5 | A-16 | 96.91 |
| Comparative Example 1 | B-1 | 34.26 |

From the results in Table 1, it is known that the photoelectric conversion devices using any of Compounds (A-1), (A-3), (A-4), (A-5) and (A-16) corresponding to the general formula (1) as a hole transport material maintained a higher retention rate even after heating for 1,000 hours, compared to the photoelectric conversion device using the standard hole transport material (B-1), and therefore have excellent heat resistance. Further, the photoelectric conversion device using Compound (A-2) as a hole transport material showed no decrease in photoelectric conversion efficiency after 1,000 hours of heating, maintaining a higher photoelectric conversion efficiency and superior heat resistance, compared to the photoelectric conversion device using the standard hole transport material.

Industrial Applicability

Using the compound represented by the general formula (1) as a hole transport material, it is possible to efficiently convert solar energy into electrical energy as a photoelectric conversion device with good photoelectric conversion efficiency and excellent heat resistance, and to provide clean energy as a solar cell. Accordingly, the industrial applicability of the invention is great.

Reference Signs List

1 Conductive Support
2 Electron Transport Layer
3 Photoelectric Conversion Layer
4 Hole Transport Layer

5 Counter Electrode

**Claims**

1. A compound represented by the following general formula (1):

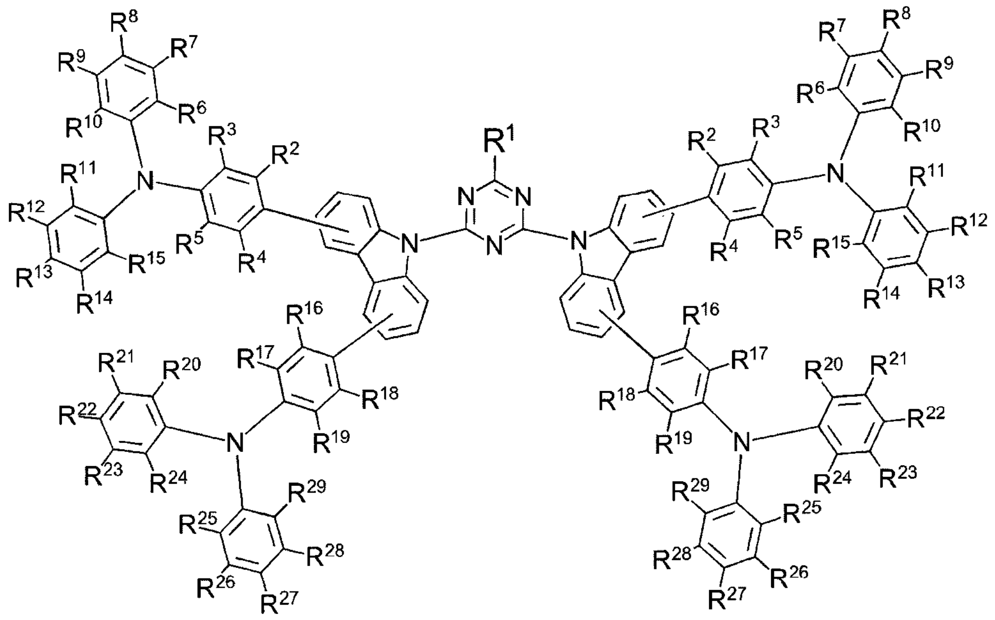

(1)

wherein:

$R^1$ represents a hydrogen atom, a halogen atom, a hydroxyl group,
a linear or branched alkyl group with 1 to 20 carbon atoms, which may have a substituent,
a linear or branched alkenyl group with 2 to 20 carbon atoms, which may have a substituent,
a linear or branched alkoxy group with 1 to 20 carbon atoms, which may have a substituent,
an aryloxy group with 6 to 30 carbon atoms, which may have a substituent,
an amino group with 0 to 60 carbon atoms, which may have a substituent,
a thio group with 0 to 20 carbon atoms, which may have a substituent,
a monovalent aromatic hydrocarbon group with 6 to 30 carbon atoms, which may have a substituent, or
a monovalent heterocyclic group with 5 to 30 ring-forming atoms, which may have a substituent,
$R^2$ to $R^{29}$ each independently represent:

a hydrogen atom,
a linear or branched alkyl group with 1 to 20 carbon atoms, which may have a substituent,
a linear or branched alkenyl group with 2 to 20 carbon atoms, which may have a substituent,
a cycloalkyl group with 3 to 12 carbon atoms, which may have a substituent,
a linear or branched alkoxy group with 1 to 20 carbon atoms, which may have a substituent,
an amino group with 0 to 20 carbon atoms, which may have a substituent,
a thio group with 0 to 20 carbon atoms, which may have a substituent, and
$R^{10}$ and $R^{11}$, and $R^{24}$ and $R^{25}$ each may bond to each other to form a ring.

2. The compound according to claim 1, wherein in the general formula (1), $R^1$ is a halogen atom, an amino group with 0 to 60 carbon atoms, which may have a substituent, an aromatic hydrocarbon group with 6 to 20 carbon atoms, which may have a substituent, or a monovalent heterocyclic group with 5 to 30 ring-forming atoms, which may have a substituent.

3. The compound according to claim 1, wherein in the general formula (1), at least one of $R^6$ to $R^{15}$ and $R^{20}$ to $R^{29}$ is a linear or branched alkoxy group with 1 to 20 carbon atoms, which may have a substituent.

4. The compound according to claim 1, wherein in the general formula (1), neither $R^{10}$ and $R^{11}$ nor $R^{24}$ and $R^{25}$ bond to each other to form a ring.

**5.** A hole transport material containing the compound described in any one of claim 1 to claim 4.

**6.** The hole transport material according to claim 5, which is for the hole transport layer of a photoelectric conversion device.

**7.** A photoelectric conversion device having a hole transport layer that contains the compound described in any one of claim 1 to claim 4.

**8.** A solar cell using the photoelectric conversion device described in claim 7.

Fig. 1

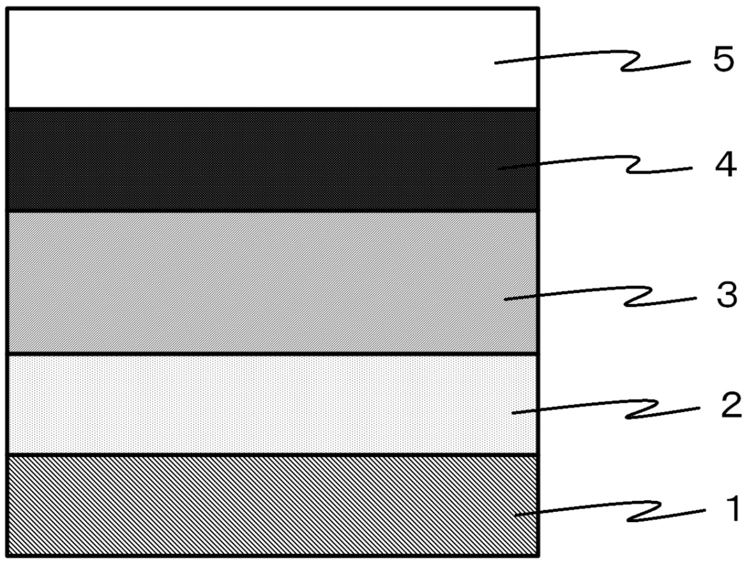

**INTERNATIONAL SEARCH REPORT**

International application No. **PCT/JP2024/012658**

**A. CLASSIFICATION OF SUBJECT MATTER**

***C07D 403/14***(2006.01)i; ***H10K 30/40***(2023.01)i; ***H10K 30/50***(2023.01)i; ***H10K 30/86***(2023.01)i; ***H10K 85/60***(2023.01)i
FI: C07D403/14 CSP; H10K30/40; H10K30/86; H10K30/50; H10K85/60

According to International Patent Classification (IPC) or to both national classification and IPC

**B. FIELDS SEARCHED**

Minimum documentation searched (classification system followed by classification symbols)

C07D403/14; H10K30/40; H10K30/50; H10K30/86; H10K85/60

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2004-171808 A (KONICA MINOLTA HOLDINGS, INC.) 17 June 2004 (2004-06-17) paragraphs [0046]-[0049], particularly, paragraph [0046], substituent group number Z12, paragraph [0047], substituent group number Z24, paragraph [0048], compound number 23 | 1-8 |
| Y | | 1-8 |
| X | CN 111548342 A (CHANGCHUN INSTITUTE OF APPLIED CHEMISTRY, CHINESE ACADEMY OF SCIENCES) 18 August 2020 (2020-08-18) claim 1, general formula, claim 3, general formula for substituents, claim 7, compound numbers 67-69 | 1-8 |
| Y | | 1-8 |
| Y | CN 106883215 A (RUISHENG TECHNOLOGY (NANJING) CO., LTD.) 23 June 2017 (2017-06-23) claims 1-11, see compound numbers: TA-1-4 to TA-1-6, TA-2-4 to TA-2-6, TA-4-4 to TA-4-6, TA-10-4, TA-14-4 to TA-14-6, TA-15-4 to TA-15-6, TA-15-34 to TA-15-36 in claim 9 | 1-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 May 2024** | **28 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**

International application No. **PCT/JP2024/012658**

**C. DOCUMENTS CONSIDERED TO BE RELEVANT**

| Category* | Citation of document, with indication, where appropriate, of the relevant passages | Relevant to claim No. |
|---|---|---|
| Y | WO 2009/104488 A1 (KONICA MINOLTA HOLDINGS, INC.) 27 August 2009 (2009-08-27)<br>paragraph [0050], compound numbers (1)-24, (1)-25, paragraphs [0054]-[0056], [0219] | 1-8 |
| Y | CN 108558739 A (SOUTH CHINA UNIVERSITY OF TECHNOLOGY) 21 September 2018 (2018-09-21)<br>claim 3 | 1-8 |
| P, A | JP 2023-072638 A (KAUNAS UNIV. OF TECHNOLOGY) 24 May 2023 (2023-05-24)<br>entire text | 1-8 |

Form PCT/ISA/210 (second sheet) (July 2022)

**INTERNATIONAL SEARCH REPORT**
**Information on patent family members**

International application No.
**PCT/JP2024/012658**

| Patent document cited in search report | Publication date (day/month/year) | Patent family member(s) | Publication date (day/month/year) |
|---|---|---|---|
| JP 2004-171808 A | 17 June 2004 | (Family: none) | |
| CN 111548342 A | 18 August 2020 | (Family: none) | |
| CN 106883215 A | 23 June 2017 | (Family: none) | |
| WO 2009/104488 A1 | 27 August 2009 | (Family: none) | |
| CN 108558739 A | 21 September 2018 | (Family: none) | |
| JP 2023-072638 A | 24 May 2023 | US 2023/0157158 A1<br>entire text<br>EP 4181225 A1<br>CN 116133444 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017104792 A


### Non-patent literature cited in the description


- *J. Am. Chem. Soc.*, 2009, vol. 131, 6050-6051
- *Science*, 2012, vol. 388, 643-647
- *Chem. Sci.*, 2019, vol. 10, 6748-6769